(19) Europäisches Patentamt

European Patent Office

Office européen des brevets

(11) **EP 2 790 566 B1**

## (12) EUROPÄISCHE PATENTSCHRIFT

(45) Veröffentlichungstag und Bekanntmachung des Hinweises auf die Patenterteilung:
**17.08.2016 Patentblatt 2016/33**

(21) Anmeldenummer: **12808681.6**

(22) Anmeldetag: **10.12.2012**

(51) Int Cl.:
*A61B 3/00* (2006.01)     *A61B 3/103* (2006.01)

(86) Internationale Anmeldenummer:
**PCT/EP2012/005092**

(87) Internationale Veröffentlichungsnummer:
**WO 2013/087187 (20.06.2013 Gazette 2013/25)**

(54) **UNIVERSELLE OBJEKTIVE REFRAKTION**

UNIVERSAL OBJECTIVE REFRACTION

RÉFRACTION OBJECTIVE UNIVERSELLE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **13.12.2011 DE 102011120973**
**24.05.2012 DE 102012010309**

(43) Veröffentlichungstag der Anmeldung:
**22.10.2014 Patentblatt 2014/43**

(73) Patentinhaber: **Rodenstock GmbH**
**80687 München (DE)**

(72) Erfinder:
• **BECKEN, Wolfgang**
**82061 Neuried (DE)**
• **ALTHEIMER, Helmut**
**87650 Baisweil-Lauchdorf (DE)**
• **ESSER, Gregor**
**80686 München (DE)**
• **WELK, Andrea**
**81547 München (DE)**

• **NÄHRING, Matthias**
**81375 München (DE)**
• **TRUMM, Stephan**
**80999 München (DE)**

(74) Vertreter: **Müller-Boré & Partner**
**Patentanwälte PartG mbB**
**Friedenheimer Brücke 21**
**80639 München (DE)**

(56) Entgegenhaltungen:
**WO-A2-2005/058136      US-A1- 2005 280 777**
**US-A1- 2009 006 508      US-A1- 2011 149 241**
**US-B1- 6 217 172**

• **SANDRA FRANCO ET AL: "Real-time dynamic monochromatic ocular wavefront aberrations during accommodation: Preliminary results", BIOENGINEERING (ENBENG), 2012 IEEE 2ND PORTUGUESE MEETING IN, IEEE, 23. Februar 2012 (2012-02-23), Seiten 1-4, XP032254872, DOI: 10.1109/ENBENG.2012.6331349 ISBN: 978-1-4673-4524-8**

**Beschreibung**

Die vorliegende Erfindung betrifft eine verbesserte objektive Refraktionsbestimmung unter Einbeziehung einer objektiven Nahrefraktion. Insbesondere betrifft die vorliegende Erfindung in einem Aspekt ein Verfahren, eine Vorrichtung sowie ein Computerprogrammprodukt für eine schnelle und dennoch verlässliche Bestimmung von individuellen Daten für zumindest ein Auge eines Brillenträgers, welche möglichst flexibel für eine individuelle Anpassung eines Brillenglases nutzbar sind.

Zur Anpassung von Brillen werden von Seiten der Brillenglashersteller immer ausgereiftere Verfahren zur individuellen Berechnung optischer Flächen unter Berücksichtigung zahlreicher individueller Daten des Brillenträgers insbesondere hinsichtlich seiner Refraktion und der individuellen Gebrauchssituation zum Einsatz gebracht. Dies bringt zwar einerseits für den Brillenträger aufgrund der besseren Anpassung der Brille bzw. der Brillengläser an die individuellen Anforderungen einen nicht unbeachtlichen Mehrwert. Dieser wird allerdings nur dann wirklich nutzbar, wenn die individuellen Daten bereits vom Optiker im Vorfeld mit ausreichender Genauigkeit bestimmt werden. Nachdem außerdem verschiedene Produkte der Brillenglashersteller oft unterschiedliche Informationen (z.B. unterschiedliche optische und/oder geometrische Daten) berücksichtigen, bedeutet es für den Optiker oft einen wesentlichen zeitlichen Aufwand und führt für den Brillenträger zu entsprechenden Unannehmlichkeiten, wenn jedesmal die erforderlichen individuellen Daten mit hinreichender Präzision erfasst werden.

Während bei einer subjektiven Refraktionsbestimmung stets die subjektive Wahrnehmung des Probanden berücksichtigt wird, erfolgen objektive Refraktionsbestimmungen aufgrund von Messungen insbesondere mittels entsprechender (vorzugsweise automatisierter) Messgeräte, insbesondere ohne dass der Proband (z.B. Brillenträger) durch Aussagen über seine subjektive Wahrnehmung Einfluss auf den Messablauf oder die Messwerte nimmt.

Das Ziel einer objektiven Refraktionsbestimmung besteht darin, so zuverlässig wie möglich diejenigen Refraktionswerte zu ermitteln, die für die Korrektur einer Fehlsichtigkeit mit einem Brillenglas erforderlich sind. Dazu wird üblicherweise z.B. ein Autorefraktor oder ein Aberrometer genutzt, um eine objektive Refraktion zu bestimmen. Herkömmlicherweise bestimmen diese Geräte die Refraktion beim Sehen in die Ferne. Dabei werden entweder die Refraktionswerte direkt bestimmt (Autorefraktor), oder aber das Gerät bestimmt intern Wellenfrontdaten inklusive der Aberrationen höherer Ordnung und bestimmt daraus dann eine Refraktion für das Sehen in die Ferne. Objektive Messungen für das Sehen in die Nähe werden dabei nicht durchgeführt, insbesondere weil die dafür nötige Akkommodation sich messtechnisch schwierig kontrollieren lässt und daher Messdaten schwierig zu interpretieren sind.

Zur Unterstützung insbesondere der objektiven der Refraktionsbestimmung beim Augenoptiker werden in zunehmendem Maße Aberrometer eingesetzt. Dabei werden im Gegensatz zu konventionellen Augenrefraktometern nicht nur die Abbildungsfehler niedriger Ordnung (insbesondere Prisma, Sphäre, Zylinder und Achslage) bestimmt, sondern auch die Abbildungsfehler höherer Ordnung (z.B. Dreiblattfehler, sphärische Aberration, Koma) erfasst. Damit trägt die Messung bereits dem Umstand Rechnung, dass manche Brillenglashersteller auch diese Abbildungsfehler höherer Ordnung bei der Optimierung einiger Brillenglasmodelle berücksichtigen. Um die damit erreichbare Verbesserung optimal nutzen zu können, wäre aber wiederum auch eine genaue Kenntnis der individuellen Größe (und vorzugsweise auch Position) der Pupille bei der jeweiligen Gebrauchssituation wünschenswert. Nachdem diese Gebrauchssituation wiederum für das jeweilige Brillenglasmodell (Lesebrille, Autofahrer-Brille, Sport-Brille) eigens bestimmt werden müsste, ist bereits erkennbar, dass die ausreichend genaue und an die individuelle Situation angepasste Bestimmung von Benutzerdaten für die Anpassung und Optimierung einer Brille mit einem wesentlichen Zeit- und Technikaufwand für den Optiker und mit entsprechenden Unannehmlichkeiten für den Brillenträger verbunden sein kann.

Die Veröffentlichung der internationalen Patentanmeldung WO2005058136 A2 offenbart eine objektive Refraktionsbestimmung anhand eines Satzes von Zernike-Koeffizienten zur Beschreibung einer Wellenfrontaberration bei einer Fernakkommodation des Auges.

Aufgabe der vorliegenden Erfindung ist es daher, eine schnelle und dennoch verlässliche Bestimmung von individuellen Daten für zumindest ein Auge eines Brillenträgers bereitzustellen, welche möglichst flexibel für eine individuelle Anpassung eines Brillenglases nutzbar sind. Insbesondere ist es Aufgabe der vorliegenden Erfindung, die objektive Refraktionsbestimmung dahingehend zu verbessern, dass damit auch Nah-Refraktionswerte (Nah-Verordnungswerte) verlässlicher ermittelt werden können. Diese Aufgabe wird insbesondere durch ein Verfahren, eine Vorrichtung und ein Computerprogrammprodukt mit den in den unabhängigen Ansprüchen 1 bzw., 9 sowie dem abhängigen Anspruch 10 angegebenen Merkmalen gelöst. Bevorzugte Ausführungsformen sind Gegenstand der entsprechenden Unteransprüche.

Somit bietet die Erfindung in einem Aspekt ein Verfahren zur objektiven Refraktionsbestimmung für ein Auge eines Brillenträgers. Dieses Verfahren umfasst ein Erfassen von Messdaten für das Auge des Brillenträgers, welche zumindest einen ersten Satz von Zernike-Koeffizienten $c_{2,F}^{0}$, $c_{2,F}^{2}$ und $c_{2,F}^{-2}$ zur Beschreibung einer Wellenfrontaberration bei einer Fernakkommodation des Auges und einem für die Nutzung des gewünschten Brillenglases relevanten

Pupillenradius $r_0$ des Auges, also einem Pupillenradius $r_0$ bei einer gewünschten Gebrauchssituation für das geplante Brillenglas, sowie einen zweiten Satz von Zernike-Koeffizienten $c_{2,N}^0$, $c_{2,N}^2$ und $c_{2,N}^{-2}$ zur Beschreibung einer Wellenfrontaberration bei einer Nahakkommodation des Auges und dem Pupillenradius $r_0$ einer Gebrauchssituation festlegen.

Jeder der Sätze von Zernike-Koeffizienten beschreibt also Abbildungsfehler des Auges anhand der Wellenfrontaberrationen, die das Auge aufgrund von Refraktionsfehlern bewirkt. Sie basieren auf tatsächlichen Messungen am Auge des Brillenträgers, welche in bekannter Weise beispielsweise mittels eines Autorefraktors oder eines Aberrometers durchgeführt werden können. Die Zernike-Koeffizienten in der Fernsicht, also die Koeffizienten des ersten Satzes, können in bekannter Weise recht zuverlässig und reproduzierbar gemessen werden, da sich insbesondere die Fernakkommodation des Auges messtechnisch vergleichsweise gut kontrollieren bzw. stimulieren lässt. Für die Messung bei einem Blick in die Nähe, also für eine Bestimmung des zweiten Satzes von Zernike-Koeffizienten, wird vorzugsweise eine möglichst starke Akkommodation des Auges an die Nahsicht stimuliert. Herkömmlich ist es zwar schwierig, den Grad der Nahakkommodation verlässlich und reproduzierbar einer bestimmten Objektentfernung bzw. einem bestimmten Akkommodationsstimulus zuzuordnen. Für das erfindungsgemäße Verfahren kommt es allerdings nicht darauf an, welchem Stimulus bzw. welcher Objektentfernung die möglichst starke Akkommodation während der Messung tatsächlich entspricht.

Vorzugsweise werden durch die Erfindung diejenigen Refraktionsdaten objektiv bestimmt, die dann bereits unmittelbar zur Optimierung und Herstellung eines Brillenglases herangezogen werden können. Insbesondere werden somit vorzugsweise die an einer Auswertungsposition für eine Brillenglasoptimierung (z.B. Scheitelpunktkugel) geltenden Werte für Sphäre, Zylinder und Achslage des Auges für einen Blick in die Nähe bei einem in der tatsächlichen Gebrauchssituation des herzustellenden Brillenglases herrschenden Pupillenradius bestimmt. Die Wellenfronten verändern sich durch Propagation und deren beste Annäherung durch Zernike-Koeffizienten hängt vom tatsächlichen Pupillenradius ab. Der erste und/oder zweite Satz von Zernike-Koeffizienten legt also vorzugsweise die Zernike-Koeffizienten an der Auswertungsposition und beim Pupillenradius $r_0$ der gewünschten Gebrauchssituation fest. Dabei ist nicht notwendigerweise erforderlich, dass die Wellenfrontaberrationen direkt an dieser Position und bei der erwarteten Helligkeit gemessen werden. Vielmehr kann die Messung der Wellenfrontaberrationen beispielsweise auch an einer anderen Position (z.B. am Hornhautscheitel) und/oder bei anderen Lichtverhältnissen erfolgen. Soweit allerdings die Lage der Auswertungsposition gegenüber der Messposition bekannt ist, sind damit ebenfalls die relevanten Zernike-Koeffizienten an der Auswertungsposition ableitbar, also (zumindest indirekt) festgelegt. Bevorzugte Implementierungen hierzu werden später noch eingehender dargelegt. Entsprechend können die Zernike-Koeffizienten beim Pupillenradius $r_0$ der gewünschten Gebrauchssituation auch aus Messungen bei anderen (vorzugsweise größeren) Pupillenradien abgeleitet werden, soweit beispielsweise die jeweiligen Pupillenradien bekannt sind. Auch hierzu werden später noch bevorzugte Implementierungen vorgestellt.

Die erfassten Messdaten müssen also den ersten und/oder zweiten Satz an Zernike-Koeffizienten nicht unmittelbar umfassen und damit "direkt" festlegen. Vielmehr können die Messdaten den ersten und/oder zweiten Satz an Zernike-Koeffizienten insoweit "indirekt" festlegen, dass diese aus den erfassten Messdaten abgeleitet werden können. Das "Erfassen von Messdaten" muss in einem erfindungsgemäßen Verfahren auch nicht unmittelbar den Vorgang des Messens umfassen. Vielmehr können die Messungen am Auge des Brillenträgers auch getrennt von der vorliegenden Erfindung (z.B. von einem Optiker oder einem Augenarzt) vorab durchgeführt und die resultierenden Ergebnisse in einer Benutzerdatenbank abgespeichert werden. Für die erfindungsgemäße objektive Refraktionsbestimmung werden die Messdaten dann beispielsweise aus dieser Datenbank oder aus einem Bestelldatensatz erfasst werden.

Der sich für die erwarteten Lichtverhältnisse in der gewünschten Gebrauchssituation ergebende Pupillenradius wird im Rahmen dieser Beschreibung gelegentlich auch als photopischer Pupillenradius bezeichnet, ohne dass die Erfindung vom Prinzip her auf bestimmte Helligkeiten der gewünschten Gebrauchssituation beschränkt wäre. Diese Terminologie soll lediglich einer Abgrenzung gegenüber bevorzugten Lichtverhältnissen während einer Wellenfrontmessung bzw. Aberrationsmessung des Auges dienen. So werden Wellenfrontmessungen vorzugsweise mit vergleichsweise wenig Licht durchgeführt, um aufgrund des sich dabei ergebenden großen Pupillenradius auch Wellenfrontkoeffizienten (Zernike-Koeffizienten) und damit Abbildungsfehler höherer Ordnung noch verlässlich messen zu können. Der in diesem Fall in der Regel größere Pupillenradius einer Messsituation könnte daher im Rahmen dieser Beschreibung auch als mesopischer Pupillenradius bezeichnet werden - wiederum ohne die Messungen auf bestimmte Lichtverhältnisse zu beschränken.

Erfindungsgemäß werden nun objektive Refraktionsdaten für Sphäre ($Sph_N^{corr}$), Zylinder ($Cyl_N^{corr}$) und Achslage ($Axis_N^{corr}$) des Auges für einen Blick in die Nähe derart ermittelt, dass die objektiven Refraktionsdaten in Abhängigkeit vom ersten und zweiten Satz von Zernike-Koeffizienten die Gleichungen

$$Sph_N^{corr} = M_N^{corr} + \sqrt{\left(J_{N,0}^{corr}\right)^2 + \left(J_{N,45}^{corr}\right)^2}$$

$$Cyl_N^{corr} = -2\sqrt{\left(J_{N,0}^{corr}\right)^2 + \left(J_{N,45}^{corr}\right)^2}$$

$$Axis_N^{corr} = \frac{1}{2}\arctan\left(-J_{N,0}^{corr}, -J_{N,45}^{corr}\right) + \frac{\pi}{2}$$

für einen korrigierten Power-Vektor $\mathbf{P}_N^{corr} = \begin{pmatrix} M_N^{corr} \\ J_{N,0}^{corr} \\ J_{N,45}^{corr} \end{pmatrix}$ für den Blick in die Nähe erfüllt, wobei der korrigierte Power-

Vektor $\mathbf{P}_N^{corr}$ in Abhängigkeit von einer Differenz

$$\Delta\mathbf{P} = \begin{pmatrix} \Delta M \\ \Delta \mathbf{J} \end{pmatrix} = \mathbf{P}_N - \mathbf{P}_F$$

zwischen einem ersten Power-Vektor

$$\mathbf{P}_F = \begin{pmatrix} M_F \\ \mathbf{J}_F \end{pmatrix} = \begin{pmatrix} -\dfrac{4\sqrt{3}}{r_0^2}c_{2,F}^0 \\ -\dfrac{2\sqrt{6}}{r_0^2}c_{2,F}^2 \\ -\dfrac{2\sqrt{6}}{r_0^2}c_{2,F}^{-2} \end{pmatrix}$$

und einem zweiten Power-Vektor

$$\mathbf{P}_N = \begin{pmatrix} M_N \\ \mathbf{J}_N \end{pmatrix} = \begin{pmatrix} -\dfrac{4\sqrt{3}}{r_0^2}c_{2,N}^0 \\ -\dfrac{2\sqrt{6}}{r_0^2}c_{2,N}^2 \\ -\dfrac{2\sqrt{6}}{r_0^2}c_{2,N}^{-2} \end{pmatrix}$$

-- dem Wert $\mathbf{P}_N - \begin{pmatrix} A_{1N} \\ 0 \\ 0 \end{pmatrix}$ entspricht, falls $\Delta M > A_{1N}$, insbesondere falls $0 \geq \Delta M > A_{1N}$; und

-- dem Wert $\mathbf{P}_F + \dfrac{A_{1N}}{\Delta M}\begin{pmatrix} 0 \\ \Delta \mathbf{J} \end{pmatrix}$ entspricht, falls $\Delta M \leq A_{1N}$, wobei $A_{1N} = -\dfrac{1}{d}$ das sphärische Äquivalent eines vorgegebenen Objektabstands $d$ für die Sicht in die Nähe ist, und wobei

$$\arctan(x, y) := \begin{cases} \arctan(y/x), & x > 0 \\ \arctan(y/x) + \pi, & x < 0, y > 0 \\ \arctan(y/x) - \pi, & x < 0, y < 0 \\ \pi/2, & x = 0, y > 0 \\ -\pi/2, & x = 0, y < 0 \end{cases} .$$

Die vorliegende Erfindung überwindet damit die Schwierigkeit bei der Interpretation herkömmlicher Nahmessungen. Hierzu beinhaltet die Erfindung eine sehr verlässliche und objektive Möglichkeit, mit der man aus einer gegebenen Wellenfront-Messung für das Sehen in die Ferne und einer gegebenen zweiten Wellenfront-Messung für das Sehen in die Nähe sowohl die Fernrefraktion als auch die Nahrefraktion ableiten kann.

Grundsätzlich sind Wellenfront-Messungen mittels z.B. Shack-Hartmann-Sensoren bekannt. Problem jeder Wellenfrontmessung ist aber immer die Akkommodation des Probanden, durch die eine objektive Messung (wie prinzipiell auch die subjektive Refraktion) zu myop ausfallen kann. Während es bisher für eine Fernmessung üblich ist, durch eine entsprechend dosierte Nebelung beim Blick auf ein Target möglichst zu erreichen, dass der Proband so gering wie möglich akkommodiert, fehlen bisher entsprechende Vorgehensweisen für eine Nahmessung.

Die vorliegende Erfindung macht nun eine entsprechende Nebelung bei der Nahmessung entbehrlich und ermöglicht dennoch, zu aussagekräftigen Nah-Messdaten zu gelangen. Dazu wird insbesondere eine Fernmessung genutzt, die Zernike-Koeffizienten für die Ferne sowie einen dazugehörigen Pupillenradius liefert. Weiter wird eine Nahmessung genutzt, die Zernike-Koeffizienten für die Nähe bei einem dazugehörigen Pupillenradius liefert. Für die Nahmessung ist dabei nur erforderlich, dass der Proband akkommodiert, während es weitgehend unerheblich ist, zu welchem Akkommodationsstimulus diese Nahmessung gehört. Weiterhin ist völlig unerheblich, wie das Gerät den Patienten dazu bringt zu akkommodieren. Natürlich ist eine Methode bevorzugt, die den Patienten dazu stimuliert, maximal möglich zu akkommodieren. Hierzu kann beispielsweise eine Abfolge an Nahmessungen stattfinden, deren Akkommodationsreiz sukzessive zunimmt, und dann wird diejenige Nahmessung selektiert, bei der die stärkste Akkommodation stattgefunden hat. Die Erfindung bietet damit ein besonders vorteilhaftes Vorgehen, von den gegebenen beiden Messungen (Ferne und Nähe) zu geeigneten Refraktionsdaten für ein Brillenglas zu gelangen.

Vorzugsweise umfasst also das Erfassen von Messdaten für das Auge des Brillenträgers ein Erfassen von ersten Messdaten für das Auge des Brillenträgers für einen Blick in die Ferne, welche den ersten Satz von Zernike-Koeffizienten festlegen; ein Erfassen einer Serie von zweiten Messdaten für das Auge des Brillenträgers für einen Blick in die Nähe bei zumindest teilweise unterschiedlicher Akkommodation des Auges; und ein Auswählen derjenigen Messdaten aus der Serie, bei denen die stärkste Akkommodation des Auges auftritt, als zweite Messdaten, welche den zweiten Satz von Zernike-Koeffizienten festlegen. Es spielt bei der erfindungsgemäßen Vorgehensweise somit keine Rolle zu welchen Entfernungen der einzelnen Akkommodationszustände der Nahsicht gehören.

Wie bereits erwähnt ist es nicht notwendig, dass die Fern- und/oder Nahmessung bei dem photopischen Pupillenradius $r_0$ durchgeführt wird. Vielmehr ist es sogar bevorzugt, wenn die Messungen der Wellenfrontaberrationen bei einem größeren Pupillenradius durchgeführt werden. So umfasst das Erfassen von Messdaten für das Auge des Brillenträgers vorzugsweise ein Erfassen von ersten Messdaten für das Auge des Brillenträgers für einen Blick in die Ferne, welche einen ersten Pupillenradius $R_F$ (also einen Pupillenradius $R_F$ bei einer ersten Messsituation) und einen ersten Satz von Zernike-Koeffizienten zur Beschreibung einer bei dem ersten Pupillenradius $R_F$ und der Fernakkommodation des Auges gemessenen Wellenfrontaberration umfassen. Vorzugsweise wird also neben einer Autorefraktor- oder einer Aberrometer-Messung auch der während dieser Messung herrschende Pupillenradius $R_F$ gemessen und gegebenenfalls in einer Benutzerdatensatz als Bestandteil der ersten Messdaten abgespeichert.

Außerdem umfasst das Erfassen von Messdaten in dieser Ausführungsform vorzugsweise in analoger Weise ein Erfassen von zweiten Messdaten für das Auge des Brillenträgers für einen Blick in die Nähe, welche einen zweiten Pupillenradius $R_N$ und einen zweiten Satz von Zernike-Koeffizienten zur Beschreibung einer bei dem zweiten Pupillenradius $R_N$ und der Nahakkommodation gemessenen Wellenfrontaberration umfassen. Dabei können die Lichtverhältnisse und/oder die Pupillenradien $R_F$ und $R_N$ für die ersten und zweiten Messdaten, also für die Fernmessung und die Nahmessung die gleichen sein.

Außerdem wird vorzugsweise unabhängig von der Fern- und/oder Nahmessung der Aberrationen der photopische Pupillenradius $r_0$ für das Auge des Brillenträgers (also der bei der Gebrauchssituation erwartete bzw. gemessene Pupillenradius $r_0$) erfasst, insbesondere gemessen bzw. aus gespeicherten individuellen Daten abgerufen. Dabei ist vorzugsweise der erste und der zweite Satz von Zernike-Koeffizienten zur Beschreibung der Wellenfrontaberrationen bei dem photopischen Pupillenradius $r_0$ des Auges durch Skalieren des ersten bzw. zweiten Satzes von Zernike-Koeffizienten zur Beschreibung der gemessenen Wellenfrontaberrationen in Abhängigkeit vom Verhältnis $\lambda_i = \dfrac{r_0}{R_i}$ des photopischen Pupillenradius $r_0$ zum ersten ($i = F$) bzw. zweiten ($i = N$) Pupillenradius $R_i$ festgelegt. Besonders bevorzugt umfassen die in den bzw. für die Messsituationen erfassten Sätze Zernike-Koeffizienten zumindest teilweise Zernike-Koeffizienten bis zur vierten Ordnung (z.B. sphärische Aberration) oder sogar teilweise bis zur sechsten Ordnung, zumindest "teilweise" heißt dabei, dass nicht notwendigerweise alle Zernike-Koeffizienten der jeweiligen Ordnung enthalten sein müssen.

In einer bevorzugten Ausführungsform umfasst der von den ersten bzw. zweiten Messdaten umfasste erste bzw. zweite Satz von Zernike-Koeffizienten zur Beschreibung der gemessenen Wellenfrontaberrationen zumindest Zernike-Koeffizienten bis zur vierten Ordnung zumindest teilweise, insbesondere zumindest die Zernike-Koeffizienten zweiter und vierter Ordnung. Dabei hängt der erste bzw. zweite Satz von Zernike-Koeffizienten zur Beschreibung der Wellenfrontaberrationen bei dem photopischen Pupillenradius $r_0$ gemäß

$$c_{2,i}^{0}\left(r_0\right) = \lambda_i^2\left(c_{2,i}^{0}\left(R_i\right) + \sqrt{15}\left(\lambda_i^2 - 1\right)c_{4,i}^{0}\left(R_i\right)\right)$$

$$c_{2,i}^{2}\left(r_0\right) = \lambda_i^2\left(c_{2}^{2}\left(R_i\right) + \sqrt{15}\left(\lambda_i^2 - 1\right)c_{4,i}^{2}\left(R_i\right)\right)$$

$$c_{2,i}^{-2}\left(r_0\right) = \lambda_i^2\left(c_{2}^{-2}\left(R_i\right) + \sqrt{15}\left(\lambda_i^2 - 1\right)c_{4,i}^{-2}\left(R_i\right)\right)$$

vom ersten ($i = F$) bzw. zweiten ($i = N$) Satz von Zernike-Koeffizienten der gemessenen Wellenfrontaberration ab. Der erste bzw. zweite Satz von Zernike-Koeffizienten ( $c_{2,i}^{0}\left(r_0\right)$ , $c_{2,i}^{2}\left(r_0\right)$ , $c_{2,i}^{-2}\left(r_0\right)$ ) zur Beschreibung der Wellenfrontaberrationen bei dem photopischen Pupillenradius $r_0$ umfasst somit insbesondere Zernike-Koeffizienten zweiter Ordnung und wird vorzugsweise durch Skalierung gemäß obigem Zusammenhang aus den Zernike-Koeffizienten zweiter und vierter Ordnung ( $c_{2,i}^{0}\left(R_i\right)$ ), $c_{2,i}^{2}\left(R_i\right)$ , $c_{2,i}^{-2}\left(R_i\right)$ , $c_{4,i}^{0}\left(R_i\right)$ , $c_{4,i}^{2}\left(R_i\right)$ , $c_{4,i}^{-2}\left(R_i\right)$ ) der Messdaten ermittelt.

In einer anderen bevorzugten Ausführungsform umfasst der von den ersten bzw. zweiten Messdaten umfasste erste bzw. zweite Satz von Zernike-Koeffizienten zur Beschreibung der gemessenen Wellenfrontaberrationen zumindest einige Zernike-Koeffizienten bis zur sechsten Ordnung, insbesondere zumindest einige der Zernike-Koeffizienten zweiter, vierter und sechster Ordnung. Dabei hängt der erste bzw. zweite Satz von Zernike-Koeffizienten zur Beschreibung der Wellenfrontaberrationen bei dem photopischen Pupillenradius $r_0$ gemäß

$$c_{2,i}^{0}\left(r_0\right) = \lambda_i^2\left(c_{2,i}^{0}\left(R_i\right) + \sqrt{15}\left(\lambda_i^2 - 1\right)c_{4,i}^{0}\left(R_i\right) + \sqrt{21}\left(\lambda_i^2 - 1\right)\left(3\lambda_i^2 - 2\right)c_{6,i}^{0}\left(R_i\right)\right)$$

$$c_{2,i}^{2}\left(r_0\right) = \lambda_i^2\left(c_{2,i}^{2}\left(R_i\right) + \sqrt{15}\left(\lambda_i^2 - 1\right)c_{4,i}^{2}\left(R_i\right) + \sqrt{21}\left(\lambda_i^2 - 1\right)\left(3\lambda_i^2 - 2\right)c_{6,i}^{2}\left(R_i\right)\right)$$

$$c_{2,i}^{-2}\left(r_0\right) = \lambda_i^2\left(c_{2,i}^{-2}\left(R_i\right) + \sqrt{15}\left(\lambda_i^2 - 1\right)c_{4,i}^{-2}\left(R_i\right) + \sqrt{21}\left(\lambda_i^2 - 1\right)\left(3\lambda_i^2 - 2\right)c_{6,i}^{-2}\left(R_i\right)\right)$$

vom ersten ($i = F$) bzw. zweiten ($i = N$) Satz von Zernike-Koeffizienten der gemessenen Wellenfrontaberration ab. Der erste bzw. zweite Satz von Zernike-Koeffizienten ($c_{2,i}^0(r_0)$ , $c_{2,i}^2(r_0)$ , $c_{2,i}^{-2}(r_0)$ ) zur Beschreibung der Wellenfrontaberrationen bei dem photopischen Pupillenradius $r_0$ umfasst somit insbesondere Zernike-Koeffizienten zweiter Ordnung und wird vorzugsweise durch Skalierung gemäß obigem Zusammenhang aus den Zernike-Koeffizienten zweiter, vierter und sechster Ordnung ($c_{2,i}^0(R_i)$, $c_{2,i}^2(R_i)$, $c_{2,i}^{-2}(R_i)$, $c_{4,i}^0(R_i)$, $c_{4,i}^2(R_i)$, $c_{4,i}^{-2}(R_i)$, $c_{6,i}^0(R_i)$, $c_{6,i}^2(R_i)$, $c_{6,i}^{-2}(R_i)$) der Messdaten ermittelt.

Wie bereits erwähnt, ist es nicht notwendig, dass die Messung der Wellenfrontaberration an derselben Position erfolgt, wie die Auswertung. So könnten die Messung der Wellenfrontaberration und ihre Darstellung durch Zernike-Polynome z.B. im Bereich des Hornhautscheitels erfolgen, während für die Optimierung und Herstellung eines Brillenglases die entsprechenden Wellenfrontaberrationen an der Scheitelpunktkugel berücksichtigt werden.

Vorzugsweise umfasst das Erfassen von Messdaten für das Auge des Brillenträgers somit ein Erfassen von ersten Messdaten für das Auge des Brillenträgers für einen Blick in die Ferne, welche einen ersten Satz von Zernike-Koeffizienten zur Beschreibung einer bei der Fernakkommodation des Auges gemessenen Wellenfrontaberration an einer ersten Messposition umfassen. Dieser Satz von Zernike-Koeffizienten kann sich wiederum entweder direkt auf den photopischen Pupillenradius oder auf einen davon abweichenden Pupillenradius beziehen, wobei im letzten Fall vorzugsweise wiederum eine Skalierung erfolgen kann.

Außerdem umfasst das Erfassen von Messdaten für das Auge des Brillenträgers vorzugsweise ein Erfassen von zweiten Messdaten für das Auge des Brillenträgers für einen Blick in die Nähe, welche einen zweiten Satz von Zernike-Koeffizienten zur Beschreibung einer bei der Nahakkommodation des Auges gemessenen Wellenfrontaberration an einer zweiten Messposition umfassen. Dabei können die erste und zweite Messposition übereinstimmen.

Das Bestimmen von objektiven Refraktionsdaten des Auges für einen Blick in die Nähe umfasst dabei vorzugsweise:

- ein Ermitteln des ersten Power-Vektors $\mathbf{P}_F(VD_F) = \begin{pmatrix} M_F(VD_F) \\ \mathbf{J}_F(VD_F) \end{pmatrix}$ aus dem von den ersten Messdaten umfassten oder gemäß einer bevorzugten Ausführungsform in Abhängigkeit von $\lambda_F = \dfrac{r_0}{R_F}$ skalierten ersten Satz von Zernike-Koeffizienten in Abhängigkeit vom Abstand $VD_F$ einer Auswertungsposition von der ersten Messposition derart, dass $M_F(VD_F)$ das sphärische Äquivalent und $J_F(VD_F)$ den zylindrischen Anteil der Wellenfrontaberration an der Auswertungsposition bei der Fernakkommodation des Auges beschreiben; und

- ein Ermitteln des zweiten Power-Vektors $\mathbf{P}_N(VD_N) = \begin{pmatrix} M_N(VD_N) \\ \mathbf{J}_N(VD_N) \end{pmatrix}$ aus dem von den zweiten Messdaten umfassten oder gemäß einer bevorzugten Ausführungsform in Abhängigkeit von $\lambda_N = \dfrac{r_0}{R_N}$ skalierten zweiten Satz von Zernike-Koeffizienten in Abhängigkeit vom Abstand $VD_N$ der Auswertungsposition von der zweiten Messposition derart, dass $M_N(VD_N)$ das sphärische Äquivalent und $J_N(VD_N)$ den zylindrischen Anteil der Wellenfrontaberration an der Auswertungsposition bei der Nahakkommodation des Auges beschreiben.

Als Auswertungsposition kann z.B. eine Position auf dem Brillenglas genutzt werden (z.B. Brillenglasrückfläche, Position auf der Scheitelpunktkugel), also insbesondere eine Position, die zur ersten und/oder zweiten Messposition einen Abstand $VD_i$ aufweist, der beispielsweise dem individuellen Hornhaut-Scheitel-Abstand des Brillenträgers für eine gewählte Brillenfassung entspricht.

In einer besonders bevorzugten Ausführungsform bietet die Erfindung somit ein Verfahren zur objektiven Refraktionsbestimmung für ein Auge eines Brillenträgers, umfassend:

- Erfassen von ersten Messdaten für das Auge des Brillenträgers für einen Blick in die Ferne, welche einen ersten Pupillenradius $R_F$ und einen ersten Satz von Zernike-Koeffizienten (zumindest bis zur 2. Ordnung) zur Beschreibung einer bei dem ersten Pupillenradius $R_F$ und einer Fernakkommodation des Auges gemessenen Wellenfrontaberration an einer ersten Messposition festlegen;
- Erfassen von zweiten Messdaten für das Auge des Brillenträgers für einen Blick in die Nähe, welche einen zweiten Pupillenradius $R_N$ und einen zweiten Satz von Zernike-Koeffizienten (zumindest bis zur 2. Ordnung) zur Beschreibung einer bei dem zweiten Pupillenradius $R_N$ und einer Nahakkommodation gemessenen Wellenfrontaberration an einer zweiten Messposition festlegen;

- Erfassen zumindest eines photopischen Pupillenradius $r_0$ für das Auge des Brillenträgers;

- Skalieren des ersten und zweiten Satzes von Zernike-Koeffizienten in Abhängigkeit vom Verhältnis $\lambda_i = \dfrac{r_0}{R_i}$ des photopischen Pupillenradius $r_0$ zum ersten $(i = F)$ bzw. zweiten $(i = N)$ Pupillenradius $R_i$ zur Beschreibung einer ersten bzw. zweiten skalierten Wellenfrontaberration für das Auge bei dem photopischen Pupillenradius an der ersten bzw. zweiten Messposition (dies führt zum entsprechenden skalierter Satz von Zernike-Koeffizienten);

- Ermitteln eines ersten Power-Vektors $\mathbf{P}_F(VD_F) = \begin{pmatrix} M_F(VD_F) \\ \mathbf{J}_F(VD_F) \end{pmatrix}$ aus dem skalierten ersten Satz von Zernike-Koeffizienten in Abhängigkeit vom Abstand $VD_F$ einer Auswertungsposition von der ersten Messposition derart, dass $M_F(VD_F)$ das sphärische Äquivalent und $J_F(VD_F)$ den zylindrischen Anteil der Wellenfrontaberration an der Auswertungsposition bei der Fernakkommodation des Auges beschreiben;

- Ermitteln eines zweiten Power-Vektors $\mathbf{P}_N(VD_N) = \begin{pmatrix} M_N(VD_N) \\ \mathbf{J}_N(VD_N) \end{pmatrix}$ aus dem skalierten zweiten Satz von Zernike-Koeffizienten in Abhängigkeit vom Abstand $YD_N$ der Auswertungsposition von der zweiten Messposition derart, dass $M_N(VD_N)$ das sphärische Äquivalent und $J_N(YD_N)$ den zylindrischen Anteil der Wellenfrontaberration an der Auswertungsposition bei der Nahakkommodation des Auges beschreiben;

- Ermitteln eines korrigierten Power-Vektors $\mathbf{P}_N^{corr}$ für den Blick in die Nähe in Abhängigkeit von einer Differenz

$$\Delta \mathbf{P} = \begin{pmatrix} \Delta M \\ \Delta \mathbf{J} \end{pmatrix} = \mathbf{P}_N(VD_N) - \mathbf{P}_F(VD_F)$$ zwischen dem ersten und zweiten Power-Vektor derart, dass der korrigierte Power-Vektor $\mathbf{P}_N^{corr}$

-- dem Wert $\mathbf{P}_N(VD_N) - \begin{pmatrix} A_{1N} \\ 0 \\ 0 \end{pmatrix}$ entspricht, falls $\Delta M > A_{1N}$, insbesondere falls $0 \geq \Delta M > A_{1N}$ ; und

-- dem Wert $\mathbf{P}_F(VD_F) + \dfrac{A_{1N}}{\Delta M}\begin{pmatrix} 0 \\ \Delta \mathbf{J} \end{pmatrix}$ entspricht, falls $\Delta M \leq A_{1N}$, wobei $A_{1N} = -\dfrac{1}{d}$ das sphärische Äquivalent eines vorgegebenen Objektabstands $d$ für die Sicht in die Nähe ist; und

- Ermitteln von objektiven Refraktionsdaten für Sphäre ($Sph_N^{corr}$), Zylinder ($Cyl_N^{corr}$) und Achslage ($Axis_N^{corr}$) des Auges für den Blick in die Nähe aus dem korrigierten Power-Vektor $\mathbf{P}_N^{corr}$ .

Insbesondere werden die Refraktionsdaten für Sphäre ($Sph_N^{corr}$), Zylinder ($Cyl_N^{corr}$) und Achslage ($Axis_N^{corr}$) des Auges für den Blick in die Nähe gemäß

$$Sph_N^{corr} = M_N^{corr} + \sqrt{\left(J_{N,0}^{corr}\right)^2 + \left(J_{N,45}^{corr}\right)^2}$$

$$Cyl_N^{corr} = -2\sqrt{\left(J_{N,0}^{corr}\right)^2 + \left(J_{N,45}^{corr}\right)^2}$$

$$Axis_N^{corr} = \frac{1}{2}\arctan\left(-J_{N,0}^{corr}, -J_{N,45}^{corr}\right) + \frac{\pi}{2}$$

aus dem korrigierten Power-Vektor $\mathbf{P}_N^{corr}$ ermittelt.

Vorzugsweise wird der erste Power-Vektor und/oder der zweite Power-Vektor derart ermittelt, dass er die Gleichung

$$\mathbf{P}_i(VD_i) = \begin{pmatrix} Sph_i(VD_i) + \dfrac{Cyl_i(VD_i)}{2} \\ -\dfrac{Cyl_i(VD_i)}{2}\cos 2\,Axis_i(VD_i) \\ -\dfrac{Cyl_i(VD_i)}{2}\sin 2\,Axis_i(VD_i) \end{pmatrix}$$

mit

$$Sph_i(VD_i) = \frac{Sph_i(0)}{1 + VD_i \times Sph_i(0)}$$

$$Cyl_i(VD_i) = \frac{Sph_i(0) + Cyl_i(0)}{1 + VD_i \times (Sph_i(0) + Cyl_i(0))} - \frac{Sph_i(0)}{1 + VD_i \times Sph_i(0)}$$

$$Axis_i(VD_i) = Axis_i(0)$$

und

$$Sph_i(0) = -\frac{4\sqrt{3}}{r_0^2}c_{2,i}^0 + \frac{2\sqrt{6}}{r_0^2}\sqrt{\left(c_{2,i}^{-2}\right)^2 + \left(c_{2,i}^2\right)^2}$$

$$Cyl_i(0) = -\frac{4\sqrt{6}}{r_0^2}\sqrt{\left(c_{2,i}^{-2}\right)^2 + \left(c_{2,i}^2\right)^2}$$

$$Axis_i(0) = \frac{1}{2}\arctan\left(c_{2,i}^2, c_{2,i}^{-2}\right) + \frac{\pi}{2}$$

für $i = F, N$ erfüllt, wobei

$$\arctan(x,y) := \begin{cases} \arctan(y/x), & x > 0 \\ \arctan(y/x) + \pi, & x < 0, y > 0 \\ \arctan(y/x) - \pi, & x < 0, y < 0 \\ \pi/2, & x = 0, y > 0 \\ -\pi/2, & x = 0, y < 0 \end{cases}.$$

und wobei $c_{2,i}^0$, $c_{2,i}^2$ und $c_{2,i}^{-2}$ die von den ersten ($i = F$) bzw. zweiten ($i = N$) Messdaten umfassten bzw. gemäß einer bevorzugten Ausführungsform skalierten Zernike-Koeffizienten zweiter Ordnung für den Blick in die Ferne ($i = F$) bzw. den Blick in die Nähe ($i = N$) bezeichnen.

In einer bevorzugten Ausführungsform umfasst das Erfassen der ersten und/oder zweiten Messdaten ein Messen von Refraktionsdaten und/oder Wellenfrontaberrationen des Auges mittels eines Autorefraktors und/oder mittels eines Aberrometers.

Vorzugsweise bietet die Erfindung ein Verfahren zum Optimierung und Herstellen eines Brillenglases für zumindest ein Auges eines Brillenträgers, umfassend:

- ein erfindungsgemäßes Verfahren zur objektiven Refraktionsbestimmung für das zumindest eine Auge des Brillenträgers insbesondere gemäß einer der hier beschriebenen bevorzugten Ausführungsformen;
- ein Optimieren oder Berechnen eines Brillenglases zur Korrektion der objektiv bestimmten Refraktion; und

- ein Herstellen des Brillenglases gemäß dem Ergebnis des Optimierens bzw. Berechnens.

In einem weiteren Aspekt bietet die Erfindung eine Vorrichtung zur objektiven Refraktionsbestimmung für ein Auge eines Brillenträgers, umfassend:

- eine Messdatenerfassungsschnittstelle zum Erfassen von Messdaten für das Auge des Brillenträgers, welche zumindest einen ersten Satz von Zernike-Koeffizienten $c_{2,F}^0$, $c_{2,F}^2$ und $c_{2,F}^{-2}$ zur Beschreibung einer Wellenfrontaberration bei einer Fernakkommodation und einem photopischen Pupillenradius $r_0$ des Auges und einen zweiten Satz von Zernike-Koeffizienten $c_{2,N}^0$, $c_{2,N}^2$ und $c_{2,N}^{-2}$ zur Beschreibung einer Wellenfrontaberration bei einer Nahakkommodation und dem photopischen Pupillenradius $r_0$ des Auges festlegen; und

- eine Refraktionsdatenermittlungseinrichtung zum Ermitteln von objektiven Refraktionsdaten für Sphäre $(Sph_N^{corr})$, Zylinder $(Cyl_N^{corr})$ und Achslage $(Axis_N^{corr})$ des Auges für einen Blick in die Nähe derart, dass die objektiven Refraktionsdaten in Abhängigkeit vom ersten und zweiten Satz von Zernike-Koeffizienten die Gleichungen

$$Sph_N^{corr} = M_N^{corr} + \sqrt{\left(J_{N,0}^{corr}\right)^2 + \left(J_{N,45}^{corr}\right)^2}$$

$$Cyl_N^{corr} = -2\sqrt{\left(J_{N,0}^{corr}\right)^2 + \left(J_{N,45}^{corr}\right)^2}$$

$$Axis_N^{corr} = \frac{1}{2}\arctan\left(-J_{N,0}^{corr}, -J_{N,45}^{corr}\right) + \frac{\pi}{2}$$

für einen korrigierten Power-Vektor $\mathbf{P}_N^{corr} = \begin{pmatrix} M_N^{corr} \\ J_{N,0}^{corr} \\ J_{N,45}^{corr} \end{pmatrix}$ für den Blick in die Nähe erfüllt, wobei der korrigierte

Power-Vektor $\mathbf{P}_N^{corr}$ in Abhängigkeit von einer Differenz

$$\Delta\mathbf{P} = \begin{pmatrix} \Delta M \\ \Delta \mathbf{J} \end{pmatrix} = \mathbf{P}_N - \mathbf{P}_F$$

zwischen einem ersten Power-Vektor

$$\mathbf{P}_F = \begin{pmatrix} M_F \\ \mathbf{J}_F \end{pmatrix} = \begin{pmatrix} -\dfrac{4\sqrt{3}}{r_0^2} c_{2,F}^0 \\ -\dfrac{2\sqrt{6}}{r_0^2} c_{2,F}^2 \\ -\dfrac{2\sqrt{6}}{r_0^2} c_{2,F}^{-2} \end{pmatrix}$$

und einem zweiten Power-Vektor

$$\mathbf{P}_N = \begin{pmatrix} M_N \\ \mathbf{J}_N \end{pmatrix} = \begin{pmatrix} -\dfrac{4\sqrt{3}}{r_0^2} c_{2,N}^0 \\ -\dfrac{2\sqrt{6}}{r_0^2} c_{2,N}^2 \\ -\dfrac{2\sqrt{6}}{r_0^2} c_{2,N}^{-2} \end{pmatrix}$$

-- dem Wert $\mathbf{P}_N - \begin{pmatrix} A_{1N} \\ 0 \\ 0 \end{pmatrix}$ entspricht, falls $\Delta M > A_{1N}$, insbesondere falls $0 \geq \Delta M > A_{1N}$ ; und

-- dem Wert $\mathbf{P}_F + \dfrac{A_{1N}}{\Delta M} \begin{pmatrix} 0 \\ \Delta \mathbf{J} \end{pmatrix}$ entspricht, falls $\Delta M \leq A_{1N}$, wobei $= A_{1N} = -\dfrac{1}{d}$ das sphärische Äquivalent eines vorgegebenen Objektabstands $d$ für die Sicht in die Nähe ist, und wobei

$$\arctan(x, y) := \begin{cases} \arctan(y/x), & x > 0 \\ \arctan(y/x) + \pi, & x < 0, y > 0 \\ \arctan(y/x) - \pi, & x < 0, y < 0 \\ \pi/2, & x = 0, y > 0 \\ -\pi/2, & x = 0, y < 0 \end{cases} .$$

Vorzugsweise umfasst die Vorrichtung Berechnungsmittel, welche ausgelegt sind, die Refraktionsdaten nach einem Verfahren zur Refraktionsbestimmung gemäß der vorliegenden Erfindung insbesondere in einer bevorzugten Ausführungsform zu ermitteln bzw. berechnen.

Im Folgenden wird zur Erläuterung des technischen Hintergrundes der Erfindung ein weiteres, beispielhaftes Verfahren zum Erfassen eines Satzes individueller Benutzerdaten für die Anpassung und Optimierung einer Brille erläutert, umfassend folgende Schritten: Zunächst umfasst das Verfahren einen Schritt des Erfassens erster aberrometrischer Daten eines ersten Auges des Brillenträgers für einen ersten Akkommodationszustand des ersten Auges bei einer ersten primären Helligkeit. Besonders bevorzugt wird als erster Akkommodationszustand eine Fernakkommodation vorgesehen. Als erste primäre Helligkeit wird vorzugsweise eine Helligkeit im Regime des mesopischen Sehens (bevorzugte Leuchtdichte im Bereich von etwa $0{,}003$ cd/m$^2$ bis etwa $30$ cd/m$^2$, besonders bevorzugt im Bereich von etwa $0{,}003$ cd/m$^2$ bis etwa $3$ cd/m$^2$, noch mehr bevorzugt im Bereich von etwa $0{,}003$ cd/m2 bis etwa $0{,}3$ cd/m$^2$, am meisten bevorzugt im Bereich von etwa $0{,}003$ cd/m$^2$ bis etwa $0{,}03$ cd/m2) vorgesehen. Als Helligkeit wird dabei insbesondere stets die am Ort des Auges bzw. die vom Auge zu erfassende Helligkeit verstanden.

Im Kontext dieser Beschreibung werden unter "aberrometrischen Daten" (bzw. "aberrometrischen Messungen") Daten zur Beschreibung der Abbildungsfehler eines Auges (Messungen zur Gewinnung dieser Daten) verstanden, deren Informationsgehalt mindestens dem Term der Ordnung "Defocus" bei Darstellung mit Zernike-Koeffizienten entspricht, idealerweise aber höhere Ordnungen (z.B. Zylinderfehler, Koma und sphärische Aberrationen) einschließt.

Nach dem Schritt des Erfassens erster aberrometrischer Daten des ersten Auges umfasst das Verfahren vorzugsweise einen Schritt des Erfassens zweiter aberrometrischer Daten des ersten Auges des Brillenträgers für einen zweiten Akkommodationszustand des ersten Auges bei einer zweiten primären Helligkeit. Dabei stimmt die zweite primäre Helligkeit vorzugsweise mit der ersten primären Helligkeit überein. Als zweite primäre Helligkeit wird vorzugsweise ebenfalls eine Helligkeit im Regime des mesopischen Sehens (bevorzugte Leuchtdichte im Bereich von etwa 0,003 cd/m$^2$ bis etwa 30 cd/m$^2$, besonders bevorzugt im Bereich von etwa 0,003 cd/m$^2$ bis etwa 3 cd/m$^2$, noch mehr bevorzugt im Bereich von etwa 0,003 cd/m$^2$ bis etwa 0,3 cd/m$^2$, am meisten bevorzugt im Bereich von etwa 0,003 cd/m$^2$ bis etwa 0,03 cd/m$^2$) vorgesehen. Besonders bevorzugt wird als zweiter Akkommodationszustand eine Nahakkommodation vorgesehen.

Dabei werden zusammen mit dem Erfassen erster aberrometrischer Daten und/oder dem Erfassen zweiter aberrometrischer Daten (also bei der ersten bzw. zweiten primären Helligkeit und beim ersten bzw. zweiten Akkommodationszustand) erste bzw. zweite primäre pupillometrische Daten für das erste Auge erfasst. Der Begriff "pupillometrische Daten" (bzw. pupillometrische Messungen) bezeichnet hier Informationen zur Größe der Pupille (bzw. Messungen zur Gewinnung dieser Daten), die mindestens eine Größenangabe (beispielsweise in Form eines Radius) umfassen aber auch die Gestalt der Pupille in komplexerer Form wiedergeben können. Zusätzlich können die pupillometrischen Daten Informationen zur Position der Pupille (beispielsweise relativ zum Hornhautscheitel oder zur optischen Achse des Auges) enthalten.

Besonders bevorzugt werden zusammen mit dem Erfassen erster aberrometrischer Daten erste primäre pupillometrische Daten für den ersten Akkommodationszustand und zusammen mit dem Erfassen zweiter aberrometrischer Daten zweite primäre pupillometrische Daten für den zweiten Akkommodationszustand erfasst.

Außerdem umfasst das Verfahren nach dem Erfassen der ersten und vorzugsweise der zweiten aberrometrischen Daten (und damit auch nach dem Erfassen von primären pupillometrischen Daten, insbesondere der ersten und zweiten primären pupillometrischen Daten) einen Schritt des Erfassens sekundärer pupillometrischer Daten für das erste Auge des Brillenträgers bei einer sekundären Helligkeit, deren Wert über dem der ersten (und vorzugsweise zweiten) primären Helligkeit liegt. Als sekundäre Helligkeit wird vorzugsweise eine Helligkeit im Regime des photopischen Sehens (bevorzugte Leuchtdichte im Bereich von etwa 3 cd/m$^2$ bis etwa 30 cd/m$^2$) vorgesehen.

Die Bezeichnungen "primär" und "sekundär" für die Helligkeiten und die pupillometrischen Daten dienen in dieser Beschreibung lediglich dem besseren Verständnis der Zuordnung der einzelnen Größen im Verfahrensablauf und haben darüber hinaus keinerlei technische Relevanz. So werden zur einfacheren Nachvollziehbarkeit der Beschreibung die zusammen mit den aberrometrischen Daten festgelegten bzw. erfassten Größen mit der zusätzlichen Bezeichnung "primär" versehen, während die nach den aberrometrischen Daten bzw. Messungen eingestellten bzw. erfassen Größen als "sekundär" bezeichnet werden.

Die für das Erfassen der pupillometrischen Daten verwendeten Helligkeiten können dabei entweder zumindest teilweise den der individuellen Gebrauchssituation entsprechenden Umständen angepasst oder unabhängig davon vorgegeben werden. In letzterem Fall lassen sich - falls dies gewünscht ist - die einer individuellen Gebrauchssituation entsprechenden Werte der individuellen Daten sehr einfach beispielsweise durch Interpolation und/oder Extrapolation insbesondere auf Basis einer angepassten analytischen Funktion ermitteln.

Damit bietet die das beispielhafte Verfahren eine sehr effiziente Möglichkeit zur Bestimmung einer universellen objektiven Refraktion mit möglichst geringem Aufwand für Anwender (z.B. Optiker) und Probanden (z.B. Kunden). Der Terminus universelle objektive Refraktion bezeichnet dabei eine objektive Refraktion, die aberrometrische Werte des gesamten Auges (d.h. der Abbildung von Objekten auf der Netzhaut) bei mindestens einem, vorzugsweise zwei Akkommodationszuständen (z.B. Ferne und Nähe) sowie pupillometrische Daten bei mindestens zwei Beleuchtungszuständen (z.B. photopisch und mesopisch) umfasst, woraus sich sehr einfach und dennoch sehr zuverlässig eine Vielzahl verschiedener Gebrauchssituation ableiten lassen.

Vorzugsweise umfasst das Verfahren außerdem nach dem Schritt des Erfassens erster aberrometrischer Daten des ersten Auges, gegebenenfalls nach dem Schritt des Erfassens zweiter aberrometrischer Daten des ersten Auges und vor dem Schritt des Erfassens sekundärer pupillometrischer Daten des ersten Auges folgende Schritte in dieser Reihenfolge:

- Erfassen erster aberrometrischer Daten eines zweiten Auges des Brillenträgers für einen ersten Akkommodationszustand des zweiten Auges bei der ersten primären Helligkeit; und
- Erfassen zweiter aberrometrischer Daten des zweiten Auges des Brillenträgers für einen zweiten Akkommodationszustand des zweiten Auges bei der zweiten primären Helligkeit.

Analog zum ersten Auge wird als erster Akkommodationszustand des zweiten Auges vorzugsweise eine Fernakkommodation und als zweiter Akkommodationszustand des zweiten Auges eine Nahakkommodation vorgesehen.

Ebenfalls analog zum ersten Auge werden in dieser bevorzugten Ausführungsform zusammen mit dem Erfassen erster aberrometrischer Daten und/oder zweiter aberrometrischer Daten des zweiten Auges (also bei der ersten bzw. zweiten primären Helligkeit) erste bzw. zweite primäre pupillometrische Daten für das zweite Auge erfasst. Außerdem umfasst das Verfahren nach dem Erfassen der ersten und zweiten aberrometrischen Daten des zweiten Auges und vor dem Erfassen sekundärer pupillometrischer Daten des ersten Auges außerdem einen Schritt des Erfassens sekundärer pupillometrischer Daten des zweiten Auges des Brillenträgers bei der sekundären Helligkeit.

Vorzugsweise entspricht der erste Akkommodationszustand des ersten und/oder zweiten Auges einer Fernakkommodation und der zweite Akkommodationszustand des ersten und/oder zweiten Auges einer Nahakkommodation. Vorzugsweise werden die Akkommodationszustände des ersten und/oder zweiten Auges durch Projizieren eines virtuellen Targets in das jeweilige Auge stimuliert. Besonders bevorzugt umfasst das Verfahren zwischen dem Erfassen der ersten und zweiten aberrometrischen Daten des ersten und/oder zweiten Auges einen Schritt des kontinuierlichen Annäherns einer virtuellen Position des virtuellen Targets an das erste bzw. zweite Auge. Damit wird in besonders zuverlässiger Weise die korrekte Akkommodation des Auges an das virtuelle Target bewirkt, was zu einer besonders zuverlässigen Messung der aberrometrischen Daten und gegebenenfalls der pupillometrischen Daten für die geringere Helligkeit (hier insbesondere auch primäre pupillometrische Daten bezeichnet) führt.

Vorzugsweise umfasst das Verfahren außerdem ein Erfassen weiterer (dritter oder sogar vierter, usw.) aberrometrischer Daten des ersten und/oder zweiten Auges des Brillenträgers für einen dritten (bzw. vierten, usw.) Akkommodationszustand des ersten bzw. zweiten Auges bei einer dritten (bzw. vierten, usw.) primären Helligkeit, wobei vorzugsweise zusammen mit dem Erfassen dritter (bzw. vierten usw.) aberrometrischer Daten des ersten bzw. zweiten Auges dritte (bzw. vierte usw.) primäre pupillometrische Daten für das erste bzw. zweite Auge erfasst werden. Vorzugsweise erfolgt dies nach dem Erfassen erster aberrometrischer Daten des ersten bzw. zweiten Auges. Besonders bevorzugt entsprechend die zweite, dritte, vierte, usw. Akkommodation unterschiedlichen Nahakkommodationen. In einem weiteren bevorzugten Aspekt sind alle primären Helligkeiten gleich.

In einer weiteren bevorzugten Ausführungsform erfolgt zusammen mit dem Erfassen der sekundären pupillometrischen Daten des ersten und/oder zweiten Auges ein Erfassen von topographischen Daten des ersten bzw. zweiten Auges bei der sekundären Helligkeit. Unter "topographischen Daten" (bzw. "topographischen Messungen") werden Daten zur Beschreibung der Topographie einer Hornhaut (bzw. Messungen zur Gewinnung dieser Daten) verstanden, deren Informationsgehalt mindestens dem Term der Ordnung "Defocus" bei Darstellung mit Zernike-Koeffizienten entspricht, idealerweise aber höhere Ordnungen (z.B. Zylinderfehler, Koma und sphärische Aberrationen) einschließt.

Nachdem die topographischen Daten zusammen mit den pupillometrischen Daten für die hohe Helligkeit (hier insbesondere auch sekundäre pupillometrische Daten bezeichnet) erfasst werden, ist keine zusätzlich Messzeit und auch keine zusätzliche Justierung des jeweiligen Auges erforderlich. Die zusätzlichen topographischen Daten stehen dann ebenfalls für eine verbesserte Optimierung eines Brillenglases zur Verfügung, ohne dass der Optiker einen erkennbaren Mehraufwand betreiben muss. Wie später noch ausgeführt, kann ein für die topographische Messung genutzter Musterprojektor gleichzeitig für die sekundäre Helligkeit sorgen, bei der die pupillometrischen Daten der höheren Helligkeit (insbesondere photopische pupillometrische Daten) erfasst werden.

Vorzugsweise umfasst das Erfassen von topographischen Daten ein Projizieren eines Lichtmusters auf das Auge und ein Erfassen von Bilddaten des Auges mittels einer einzigen Bilderfassungseinrichtung (Kamera), aus denen sowohl die von dem projizierten Lichtmuster erzeugten Lichtreflexionen zur Auswertung der topographischen Daten als auch die pupillometrischen Daten ermittelt werden. In einer bevorzugten Ausführungsform umfasst die Bilderfassungseinrichtung eine Farbkamera, wobei das projizierte Lichtmuster farblich von der in den Bilddaten erfassten Pupille unterscheidbar ist. Damit kann eine einfachere Auswertung der pupillometrischen Daten und der topographischen Daten aus demselben Bilddatensatz erreicht werden. Alternativ können auch zwei verschiedene Kameras für die Erfassung der topographischen Daten und die pupillometrischen Daten vorgesehen sein, die beispielsweise verschiedene Farbfilter aufweisen, um wiederum eine einfachere Auswertung der jeweiligen Bildelemente zu erreichen.

Ferner wird im Folgenden zur Erläuterung des technischen Hintergrundes eine weitere, beispielhafte Vorrichtung zum Erfassen eines Satzes individueller Benutzerdaten für die Anpassung und Optimierung einer Brille beschrieben, wobei die Vorrichtung umfasst:

- eine Aberrometereinrichtung zum Erfassen aberrometrischer Daten zumindest eines Auges des Brillenträgers zumindest für einen ersten Akkommodationszustand des Auges bei einer ersten primären Helligkeit und vorzugsweise für einen zweiten Akkommodationszustand des Auges bei einer zweiten primären Helligkeit;
- eine Beleuchtungseinrichtung zum Erzeugen einer sekundären Helligkeit, welche größer ist als die erste (und vorzugsweise zweite) primäre Helligkeit; und
- eine Pupillometereinrichtung, welche ausgelegt ist, erste primäre pupillometrische Daten des zumindest einen Auges bei der ersten primären Helligkeit und/oder zweite primäre pupillometrische Daten des zumindest einen Auges bei der zweiten primären Helligkeit zu erfassen und sekundäre pupillometrische Daten des zumindest einen Auges bei der sekundären Helligkeit zu erfassen,

wobei die Vorrichtung ausgelegt ist, ein Verfahren gemäß der vorliegenden Erfindung insbesondere in einer der hier beschriebenen bevorzugten Ausführungsformen auszuführen.

Vorzugsweise umfasst die Aberrometereinrichtung eine Akkommodationsstimulanzeinrichtung, welche ausgelegt ist, ein virtuelles Target in das zumindest eine Auge zu projizieren und die virtuelle Position des virtuellen Targets zwischen einer Position zur Stimulation einer Fernakkommodation und einer Position zur Stimulation einer Nahakkommodation zu verändern. Als virtuelles Target wird insbesondere eine optische Projektion in das Auge des Probanden derart angesehen, dass diese Projektion auf der Netzhaut des Auges ein Abbild erzeugt, das dem Abbild eines realen Objekt in einer bestimmten Entfernung vom Auge entspricht. Diese bestimmte Entfernung wird für das virtuelle Target hier auch als virtuelle Position bezeichnet. Da es sich insbesondere nicht um ein reales Objekt handelt, kann durch geeignete Konstruktion des optischen Systems zur Projektion auch eine virtuelle Position jenseits von unendlich simuliert werden.

Vorzugsweise umfasst die Vorrichtung:

- einen Musterprojektor zur Projektion eines Lichtmusters auf das zumindest eine Auge (insbesondere auf die Hornhaut des zumindest einen Auges); und
- eine Topographie-Auswerteeinrichtung, welche ausgelegt ist, aus Reflexionen des Lichtmusters am Auge (insbesondere an der Hornhaut des Auges) topographische Daten zu ermitteln.

In einer bevorzugten Ausführungsform dient der Musterprojektor gleichzeitig als die bereits oben beschriebene Beleuchtungseinrichtung zum Erzeugen der sekundären Helligkeit.

Neben entsprechenden Verfahren insbesondere zur Refraktionsbestimmung vorzugsweise unter Einbeziehung einer oder mehrerer der als funktionale Abläufe in den erfindungsgemäßen Vorrichtungen implementierten entsprechenden Verfahrensschritten bietet die Erfindung auch ein Computerprogrammprodukt, insbesondere in Form eines Speichermediums oder einer Signalfolge, umfassend computerlesbare Anweisungen, welche wenn geladen in einen Speicher eines Computers, vorzugsweise im Speicher einer Datenverarbeitungseinheit einer Vorrichtung gemäß der vorliegenden Erfindung, und ausgeführt von dem Computer, welcher insbesondere ausgelegt ist eine erfindungsgemäße Vorrichtung zu steuern oder zu kontrollieren oder von einer erfindungsgemäßen Vorrichtung umfasst ist, bewirken, dass der Computer (und damit insbesondere die erfindungsgemäße Vorrichtung) ein Verfahren gemäß der vorliegenden Erfindung, insbesondere in einer bevorzugten Ausführungsform durchführt bzw. steuert.

Die Erfindung wird nachfolgend mit Verweis auf begleitende Zeichnungen anhand bevorzugter Ausführungsformen beispielhaft beschrieben. Dabei zeigt:

Fig. 1    ein Flussdiagramm zur schematischen Veranschaulichung eines Verfahrensablaufs gemäß einer bevorzugten Ausführungsform der Erfindung.

Fig. 2    eine schematische Darstellung eines Ablaufs eines Verfahren gemäß einer bevorzugten Ausführungsform der vorliegenden Erfindung.

In einer bevorzugten Ausführungsform werden die Zernike-Koeffizienten für den Blick in die Ferne und den Blick in die Nähe aus einem dem Datensatz abgerufen, der für den Brillenträger bereitgestellt wurde. Dieser Datensatz kann in gesonderten Messungen erstellt und für die weitere Verarbeitung und Auswertung oder für eine Bestellung einer Brille gespeichert worden sein. So kann beispielsweise ein Optiker den Datensatz durch Messung von Wellenfronten am Auge des Brillenträgers erstellen und zur weiteren Verarbeitung, insbesondere zur Nutzung für ein erfindungsgemäßes Verfahren an einen Brillenglashersteller oder ein optisches Rechenbüro übertragen werden. Es ist auch möglich, dass bereits der Optiker ein entsprechendes Computersystem zur Verfügung hat, welches in der erfindungsgemäßen Weise auf Basis des für den Brillenträger eventuell in einer gesonderten Messung ermittelten Datensatzes korrigierte Nah-Refraktionsdaten ermittelt, welche beispielsweise anschließend an einen Brillenglashersteller übermittelt werden können. Andererseits ist es auch möglich, dass Nah- und/oder Fernrefraktionsdaten bzw. Zernike-Koeffizienten für Nah- und Fernsicht nach der Messung unmittelbar in einer erfindungsgemäßen Weise zur (objektiven) Refraktionsbestimmung weiterverarbeitet werden, insbesondere ohne vorher in einer Benutzerdatenbank abgelegt zu werden.

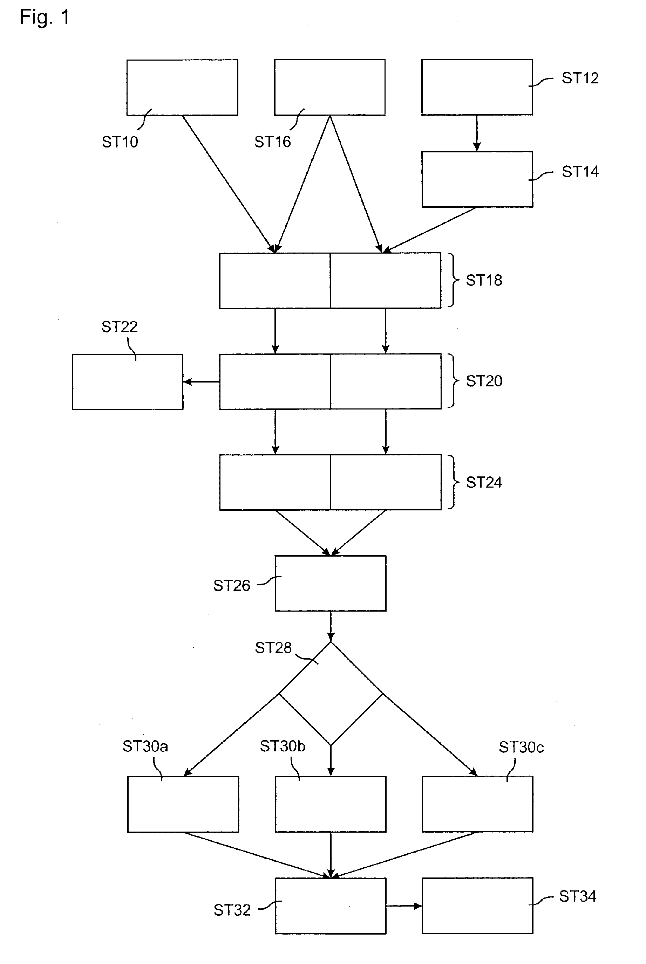

Unabhängig davon, wie die Messwerte bzw. Messdaten für eine erfindungsgemäße Refraktionsbestimmung erfasst werden, umfasst die in Fig. 1 dargestellte bevorzugte Ausführungsform einen Schnitt ST10 des Erfassens von ersten Messdaten, welche insbesondere auf einer herkömmlichen objektiven Refraktionsbestimmung für eine Sicht in die Ferne, also bei möglichst geringer Akkommodation des Auges, beruhen können. In einer bevorzugten Ausführungsform umfassen die Messdaten eine Vielzahl von Zernike-Koeffizienten, welche unter mesopischen Bedingungen und einem daraus resultierenden Pupillenradius $R_0$ gemessen wurden. Die ersten Messdaten umfassen daher insbesondere den Pupillenradius, der vorzugsweise ebenfalls während der objektiven Refraktionsbestimmung gemessen wird oder wurde, sowie einen Satz von Zernike-Koeffizienten $c_{n,F}^{m}\left(R_{0}\right)$ (erster Satz von Zernike-Koeffizienten), welche die Wellenfrontaberration z.B. am Hornhautscheitel bei einer Fernakkommodation des Auges beschreiben.

Anlog dazu werde in einem Schritt ST12 Messdaten für eine Nahakkommodation des Auges erfasst bzw. gemessen. Hierzu kann beispielsweise eine Abfolge (Serie) an Nahmessungen stattfinden, deren Akkommodationsreiz sukzessive zunimmt. Anschließend wird dann in Schritt ST14 diejenige Nahmessung selektiert, bei der stärkste Akkommodation stattgefunden hat, also wie später in Gleichung (9b) beschrieben $\Delta M$ den negativsten Wert aufweist. Die in den Schritten ST12 und ST14 erfassten Messdaten umfassen somit vorzugsweise den Pupillenradius $R_{0}$ während der Messung sowie einen Satz von Zernike-Koeffizienten $c_{n,N}^{m}\left(R_{0}\right)$ (zweiter Satz von Zernike-Koeffizienten), welche die Wellenfrontaberration z.B. am Hornhautscheitel bei einer Nahakkommodation des Auges beschreiben. Welcher Akkommodationsreiz bzw. welche Objektentfernung zur maximalen Akkommodation geführt hat spielt dabei keine wesentliche Rolle.

Soweit nachfolgende Ausführungen in analoger Weise auf die Fern- und die Nahakkommodation anwendbar sind, kann in den Formeln auf den entsprechenden Index ("*F*" für Fern und "*N*" für Nah) verzichtet werden.

Falls die in den erfassten Messdaten enthaltenen Zernike-Koeffizienten für einen (mesopischen) Pupillenradius $R_{0}$ als $c_{n}^{m}\left(R_{0}\right)$ gegeben sind, dann besitzt der konzentrische Ausschnitt der gleichen Wellenfront für einen (photopischen) Pupillenradius $r_{0} < R_{0}$ Zernike-Koeffizienten $c_{n}^{m}\left(r_{0}\right)$, die aus den $c_{n}^{m}\left(R_{0}\right)$ und dem Radienverhältnis $\lambda = r_{0}/R_{0}$ vorzugsweise mittels der Skalierungsrelation

$$c_{2}^{0}(r_{0}) = \lambda^{2}\left(c_{2}^{0}(R_{0}) + \sqrt{15}\left(\lambda^{2}-1\right)c_{4}^{0}(R_{0}) + \sqrt{21}\left(\lambda^{2}-1\right)\left(3\lambda^{2}-2\right)c_{6}^{0}(R_{0})\right)$$
$$c_{2}^{2}(r_{0}) = \lambda^{2}\left(c_{2}^{2}(R_{0}) + \sqrt{15}\left(\lambda^{2}-1\right)c_{4}^{2}(R_{0}) + \sqrt{21}\left(\lambda^{2}-1\right)\left(3\lambda^{2}-2\right)c_{6}^{2}(R_{0})\right) \qquad (0)$$
$$c_{2}^{-2}(r_{0}) = \lambda^{2}\left(c_{2}^{-2}(R_{0}) + \sqrt{15}\left(\lambda^{2}-1\right)c_{4}^{-2}(R_{0}) + \sqrt{21}\left(\lambda^{2}-1\right)\left(3\lambda^{2}-2\right)c_{6}^{-2}(R_{0})\right)$$

abgeleitet werden können. Dazu umfasst das Verfahren zunächst vorzugsweise einen Schritt ST16 des Erfassens bzw. Messens eines vorzugsweise individuellen photopischen Pupillenradius $r_{0}$. Vorzugsweise wird in einem Schritt ST18 des Skalierens der Zernike-Koeffizienten sowohl für die Fernsicht als auch für die Nahsicht die Relation gemäß Gleichung (0) genutzt, um eine Messung, die zu einer (großen) mesopischen Pupille gehören, auf eine (kleinere) photopische Referenzpupille herunterzuskalieren.

Zur weiteren Beschreibung werden vorzugsweise Power-Vektoren

$$\mathbf{P} = \begin{pmatrix} M \\ J_{0} \\ J_{45} \end{pmatrix} \qquad (1)$$

genutzt. Zur späteren Vereinfachung wird der zylindrische Teil von P als

$$\mathbf{J} = \begin{pmatrix} J_{0} \\ J_{45} \end{pmatrix} \qquad (1a)$$

zusammengefasst, so dass sich der Power-Vektor auch in der Form

$$\mathbf{P} = \begin{pmatrix} M \\ \mathbf{J} \end{pmatrix} \qquad (1b)$$

schreiben lässt.

Um einen Zusammenhang zwischen Zernike-Koeffizienten und SZA-Werten (Refraktionsdaten für Sphäre, Zylinder, Achse) herzustellen, die in derselben Referenzebene (also derselben Auswertungsposition) gelten sollen, werden nun vorzugsweise folgende Formeln angewandt, wonach der Power-Vektor

$$\mathbf{P}(Sph, Cyl, Axis) = \begin{pmatrix} Sph + \dfrac{Cyl}{2} \\ -\dfrac{Cyl}{2}\cos 2Axis \\ -\dfrac{Cyl}{2}\sin 2Axis \end{pmatrix} \qquad \mathbf{P}(c_2^0, c_2^{-2}, c_2^2) = \begin{pmatrix} -\dfrac{4\sqrt{3}}{r_0^2}c_2^0 \\ -\dfrac{2\sqrt{6}}{r_0^2}c_2^2 \\ -\dfrac{2\sqrt{6}}{r_0^2}c_2^{-2} \end{pmatrix} \qquad (2)$$

entweder als Funktion der Refraktionsdaten *Sph*, *Cyl*, *Axis* oder als Funktion der Zernike-Koeffizienten $c_2^0, c_2^2, c_2^{-2}$ betrachtet werden kann, wobei $r_0$ der Pupillenradius ist. Hierzu wird auch auf Seite 334 in "Adaptive Optics for Vision Science", Porter et al., Wiley 2006 verwiesen. Daraus ergibt sich

$$Sph = -\frac{4\sqrt{3}}{r_0^2}c_2^0 + \frac{2\sqrt{6}}{r_0^2}\sqrt{\left(c_2^{-2}\right)^2 + \left(c_2^2\right)^2}$$

$$Cyl = -\frac{4\sqrt{6}}{r_0^2}\sqrt{\left(c_2^{-2}\right)^2 + \left(c_2^2\right)^2} \qquad (3a)$$

$$Axis = \frac{1}{2}\arctan\left(c_2^2, c_2^{-2}\right) + \frac{\pi}{2}$$

$$Sph = M + \sqrt{J_0^2 + J_{45}^2}$$

$$Cyl = -2\sqrt{J_0^2 + J_{45}^2} \qquad (3b)$$

$$Axis = \frac{1}{2}\arctan\left(-J_0, -J_{45}\right) + \frac{\pi}{2}$$

wobei

$$\arctan(x, y) := \begin{cases} \arctan(y/x), & x > 0 \\ \arctan(y/x) + \pi, & x < 0, y > 0 \\ \arctan(y/x) - \pi, & x < 0, y < 0 \\ \pi/2, & x = 0, y > 0 \\ -\pi/2, & x = 0, y < 0 \end{cases}$$

Die Gleichungen (3a) und (3b) sind äquivalent zu den Gleichungen (2) und liefern *Sph,Cyl,Axis* für gegebene Zernike-Koeffizienten in der gleichen Referenz-Ebene und gegebenen Pupillenradius.

Falls sich die Messposition(en) (z.B. die Ebene des Hornhautscheitels) von der interessierenden Ebene bzw. der Auswertungsposition (z.B. die Brillenglasebene oder die Scheitelpunktkugel) unterscheidet und von dieser um einen Hornhaut-Scheitel-Abstand *VD* entfernt liegt, dann werden die propagierten Refraktionsdaten vorzugsweise bestimmt durch

$$Sph(VD) = \frac{Sph(0)}{1 + VD \times Sph(0)}$$

$$Cyl(VD) = \frac{Sph(0) + Cyl(0)}{1 + VD \times \left(Sph(0) + Cyl(0)\right)} - \frac{Sph(0)}{1 + VD \times Sph(0)} \qquad (4)$$

$$Axis(VD) = Axis(0)$$

Entsprechend umfasst das Verfahren vorzugsweise einen Schritt ST20 des Ermitteins bzw. Berechnens propagierter Refraktionswerte, also der Refraktionswerte für Sphäre, Zylinder und Achslage von propagierten Wellenfronten, aus den Refraktionswerten an den Messpositionen, welche insbesondere aus den ursprünglichen bzw. den skalierten Zernike-Koeffizienten gemäß Gleichung (3a) ermittelt werden.

Um die objektive Refraktion zu bestimmen liefert das gesamte Verfahren vorzugsweise objektive Refraktionswerte sowohl für die Fernsicht als auch für die Nahsicht. Dabei werden die Schritte ST18 des Skalierens der Zernike-Koeffizienten und ST20 des Ermitteins propagierter Refraktionswerte sowohl für die ersten Messdaten (Fernsicht) als auch für die zweiten Messdaten (Nahsicht) durchgeführt.

Vorzugsweise werden die gemessenen Zernike-Koeffizienten der Fernmessung, die in 2. Ordnung durch $c_{2,F}^0(R_{0,F}), c_{2,F}^2(R_{0,F}), c_{2,F}^{-2}(R_{0,F})$ gegeben sind können, zuerst in Schritt ST18 zu den Werten $c_{2,F}^0(r_0), c_{2,F}^2(r_0), c_{2,F}^{-2}(r_0)$ beim photopischen Pupillenradius mittels Gleichung (0) herunterskaliert mit $\lambda = r_0/R_{0,F}$. Dabei ist $R_{0,F}$ der gemessene mesopische Pupillenradius, der zu den Daten der Fernmessung gehört, während $r_0$ der photopische Pupillenradius ist, der aus einer separaten Messung ST16 stammen kann.

Die im Schritt ST18 resultierenden skalierten Zernike-Koeffizienten werden dann zu Refraktionsdaten $Sph_F(0)$, $Cyl_F(0)$, $Axis_F(0)$ für die Ferne mittels Gleichung (3a) umgewandelt, wobei in Gleichung (3a) die Zernike-Koeffizienten $c_2^0, c_2^2, c_2^{-2}$ durch die photopischen Zernike-Koeffizienten $c_{2,F}^0(r_0), c_{2,F}^2(r_0), c_{2,F}^{-2}(r_0)$ für die Ferne ersetzt werden, die vorzugsweise gemäß Gleichung (0) bestimmt werden.

Die Fern-Refraktionsdaten $Sph_F(0)$, $Cyl_F(0)$, $Axis_F(0)$ werden in Schritt ST20 vorzugsweise anhand von Gleichung (4) transformiert, um die an der Auswertungsposition gültigen Refraktionsdaten $Sph_F(VD)$, $Cyl_F(VD)$, $Axis_F(VD)$ im Abstand $VD$ von der Messposition zu erhalten. In Schritt ST22 werden dann die propagierten Fern-Refraktionsdaten $Sph_F(VD)$, $Cyl_F(VD)$, $Axis_F(VD)$ als objektive Refraktionswerte für die Fernsicht ausgegeben.

Für die Bestimmung der Nah-Refraktionswerte umfasst das erfindungsgemäße Verfahren insbesondere in der in Fig. 1 veranschaulichten bevorzugten Ausführungsform vorzugsweise zunächst analoge Schritte wie für die Fern-Refraktionswerte. Da zur Interpretation der Nahmessung die Daten der Fernmessung auch relevant sind, werden sowohl Nah- als auch Fern-Refraktionsdaten aus den vorhandenen Zernike-Koeffizienten bestimmt. Hinsichtlich der Fernrefraktion können hier für die Ergebnisse $Sph_F(VD)$, $Cyl_F(VD)$, $Axis_F(VD)$ wie oben beschrieben benutzt werden. Für die Nahmessung werden vorzugsweise zunächst analoge Schritte durchgeführt.

Vorzugsweise werden die gemessenen Zernike-Koeffizienten der Nahmessung, die in 2. Ordnung durch $c_{2,N}^0(R_{0,N}), c_{2,N}^2(R_{0,N}), c_{2,N}^{-2}(R_{0,N})$ gegeben sein können, zuerst in Schritt ST18 zu den Werten $c_{2,N}^0(r_0), c_{2,N}^2(r_0), c_{2,N}^{-2}(r_0)$ beim photopischen Pupillenradius mittels Gleichung (0) herunterskaliert mit $\lambda = r_0/R_{0,N}$. Dabei ist $R_{0,N}$ der gemessene mesopische Pupillenradius, der zu den Daten der Nahmessung gehört, während $r_0$ der photopische Pupillenradius ist, der aus einer separaten Messung ST16 stammen kann.

Die im Schritt ST18 resultierenden skalierten Zernike-Koeffizienten werden dann zu Refraktionsdaten $Sph_N(0)$, $Cyl_N(0)$, $Axis_N(0)$ für die Nähe mittels Gleichung (3a) umgewandelt, wobei in Gleichung (3a) die Zernike-Koeffizienten $c_2^0, c_2^2, c_2^{-2}$ durch die photopischen Zernike-Koeffizienten $c_{2,N}^0(r_0), c_{2,N}^2(r_0), c_{2,N}^{-2}(r_0)$ für die Nähe ersetzt werden, die vorzugsweise gemäß Gleichung (0) ermittelt werden.

Die Nah-Refraktionsdaten $Sph_N(0)$, $Cyl_N(0)$, $Axis_N(0)$ werden in Schritt ST20 vorzugsweise anhand von Gleichung (4) transformiert, um die an der Auswertungsposition gültigen Refraktionsdaten $Sph_N(VD)$, $Cyl_N(VD)$, $Axis_N(VD)$ im Abstand $VD$ von der Messposition zu erhalten.

Im einem nachfolgenden Schritt ST24 werden vorzugsweise die propagierten Fern-Refraktionsdaten $Sph_F(VD)$, $Cyl_F(VD)$, $Axis_F(VD)$ mittels Gleichung (2) in Power-Vektoren transformiert, d.h.

$$\mathbf{P}_F(VD) = \mathbf{P}\big(Sph_F(VD), Cyl_F(VD), Axis_F(VD)\big) \tag{8a}$$

und analog wird der Power-Vektor gebildet, der zu den propagierten Nah-Refraktionsdaten $Sph_N(VD)$, $Cyl_N(VD)$, $Axis_N(VD)$ gehört, durch

$$\mathbf{P}_N(VD) = \mathbf{P}\big(Sph_N(VD), Cyl_N(VD), Axis_N(VD)\big) \tag{8b}$$

Um die Nahmessungs-Daten nun zu interpretieren, wird im Schritt ST26 im Power-Vektor-Raum die Differenz zwischen Fernmessungs-Daten und Nahmessungs-Daten, also der Differenz-Powervektor

$$\Delta\mathbf{P} = \mathbf{P}_N(VD) - \mathbf{P}_F(VD) \tag{9a}$$

gebildet. Separat in sphärischen und zylindrischen Komponenten geschrieben entspricht das

$$\begin{aligned} \Delta M &= M_N(VD) - M_F(VD) \\ \Delta\mathbf{J} &= \mathbf{J}_N(VD) - \mathbf{J}_F(VD) \end{aligned} \tag{9b}$$

Der Power-Vektor in Gleichung (8b) beschreibt primär die Eigenschaften der Wellenfront bei der Messung. Um davon zu Refraktionsdaten zu gelangen, die als Korrektion für ein Brillenglas maßgeblich sind, wird nun noch berücksichtigt, dass das Brillenglas im Nahteil nicht beim Sehen in die Ferne unterstützen soll, sondern beim Sehen in die Nähe, bevorzugt beim Sehen bei einem Objektabstand, der bevorzugt bei $A_{1N}$ = -2.5$dpt$ liegt, was einem Objektabstand von 40 cm entspricht. Der Power-Vektor wird gegenüber Gleichung (8b) noch um Beiträge korrigiert, die vom Objektabstand abhängen. Um einen korrigierten Power-Vektor $\mathbf{P}_N^{corr}(VD)$ der Nahmessung zu bilden, werden in Schritt ST28 eines Vergleichs der sphärischen Komponente des Differenz-Power-Vektors vorzugsweise drei Fälle unterschieden:

a)  $\Delta M > 0$

In diesem Fall ist die Akkommodation während der Nahmessung sogar geringer als während der Fernmessung. Dies weist beispielsweise darauf hin, dass entweder zumindest eine der beiden Messungen nicht vertrauenswürdig ist, oder dass das Rauschen in den Messdaten den Fall $\Delta M > 0$ verursacht hat. In einer bevorzugten Ausführungsform wird daher im Fall $\Delta M > 0$ in Schritt ST30a eine der beiden Messungen verworfen und durch die andere ersetzt. Besonders bevorzugt wird in diesem Fall die ursprüngliche Nahmessung ersetzt durch

$$\mathbf{P}_N^{corr}(VD) = \mathbf{P}_F(VD) - \begin{pmatrix} A_{1N} \\ 0 \\ 0 \end{pmatrix} \tag{10}$$

In einer anderen bevorzugten Ausführungsform werden in Schritt ST30a die beiden Messungen vertauscht. In dieser Ausführungsform werden die korrigierten Power-Vektoren daher bestimmt durch:

$$\mathbf{P}_F^{corr}(VD) = \mathbf{P}_N(VD)$$

$$\mathbf{P}_N^{corr}(VD) = \mathbf{P}_F(VD) - \begin{pmatrix} A_{1N} \\ 0 \\ 0 \end{pmatrix} \tag{10a}$$

b)  $A_{1N} < \Delta M \le 0$, wobei vorzugsweise $A_{1N}$ = -2.5$dpt$

In diesem Fall findet eine geringfügige Akkommodation statt, aber nicht genug, um bei einem verlangten Objektabstand, der vorzugsweise bei $A_{1N}$ = -2.5$dpt$ liegt, scharf zu sehen. Dann benötigt die korrigierte sphärische Komponente des Brillenglases eine Addition, die durch den korrigierten Nahwert

$$M_N^{corr}(VD) = M_N(VD) - A_{1N} \qquad (11a)$$

gegeben ist, während des zylindrischen Komponenten unverändert bleiben:

$$\mathbf{J}_N^{corr}(VD) = \mathbf{J}_N(VD) \qquad (11b)$$

Somit lautet der in Schritt ST30b ermittelte korrigierte Nah-Power-Vektor vorzugsweise

$$\mathbf{P}_N^{corr}(VD) = \begin{pmatrix} M_N^{corr}(VD) \\ \mathbf{J}_N^{corr}(VD) \end{pmatrix} = \mathbf{P}_N(VD) - \begin{pmatrix} A_{1N} \\ 0 \\ 0 \end{pmatrix} \qquad (11)$$

c) $\quad \Delta M \leq A_{1N}$

In diesem Fall ist genügend Akkommodation vorhanden, um jede Anforderung an das Nahsehen ohne eine sphärische Addition im Brillenglas zu erfüllen. Daher wird vorzugsweise die korrigierte sphärische Komponente der Nahsicht gleich der Fernmessung gesetzt:

$$M_N^{corr}(VD) = M_F(VD) \qquad (12a)$$

Die zylindrische Komponente der Messung jedoch entspricht einer Akkommodation von - $\Delta M$, obwohl der interessierende Anteil ja nur einer Akkommodation von - $A_{1N}$ entsprechen würde. Daher wird die zylindrische Komponente vorzugsweise gemäß

$$\mathbf{J}_N^{corr}(VD) = \mathbf{J}_F(VD) + \frac{A_{1N}}{\Delta M}\Delta\mathbf{J} \qquad (12b)$$

zurückskaliert, so dass der in Schritt ST30c ermittelte korrigierte Nah-Power-Vektor vorzugsweise gemäß

$$\mathbf{P}_N^{corr}(VD) = \begin{pmatrix} M_N^{corr}(VD) \\ \mathbf{J}_N^{corr}(VD) \end{pmatrix} = \mathbf{P}_F(VD) + \frac{A_{1N}}{\Delta M}\begin{pmatrix} 0 \\ \Delta\mathbf{J} \end{pmatrix} \qquad (12)$$

bestimmt wird.

Der korrigierte Nah-Powervektor wird schließlich in Schritt ST32 zurücktransformiert zu korrigierten Nah-Refraktionsdaten $Sph_N^{corr}(VD), Cyl_N^{corr}(VD), Axis_N^{corr}(VD)$ vorzugsweise entsprechend der Gleichung (3b). In Schritt ST34 werden dann die korrigierten Nah-Refraktionsdaten $Sph_N^{corr}(VD), Cyl_N^{corr}(VD), Axis_N^{corr}(VD)$ als objektive Refraktionswerte für die Nahsicht ausgegeben.

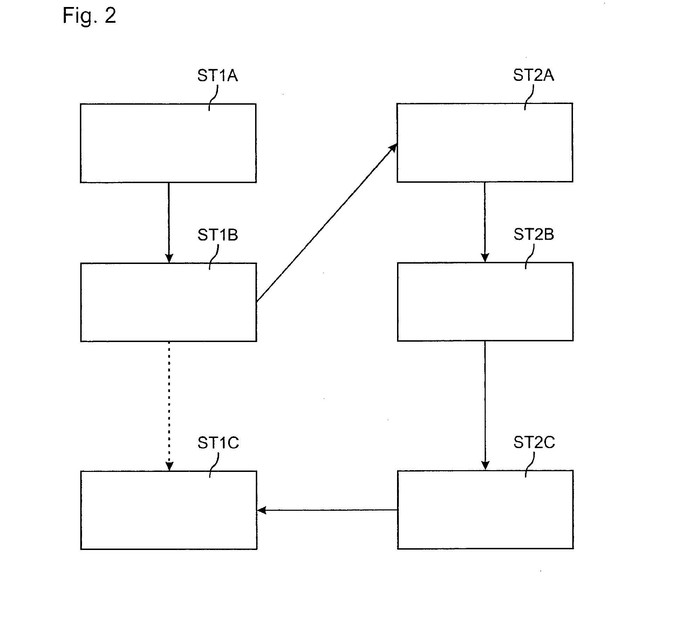

In einem weiteren Aspekt, welcher vorzugsweise zusammen mit einem oder mehreren der oben beschriebenen Aspekte oder eigenständig genutzt werden kann, wird zunächst eine ganz spezielle Sequenz von Verfahrensschritten gemäß einer bevorzugten Ausführungsform beschrieben, deren einzelne Verfahrensschritte in den nachfolgenden Abschnitten genauer beschrieben werden. In dieser bevorzugten Ausführungsform, wie sie in Fig. 2 veranschaulicht ist, umfasst ein Verfahren folgende Schritte in dieser Reihenfolge:

- ST1A:     Ermittlung aberrometrischer Daten des ersten Auges für die Fernsicht
- ST1B:     Ermittlung aberrometrischer Daten des ersten Auges für die Nahsicht
- ST2A:     Ermittlung aberrometrischer Daten des zweiten Auges für die Fernsicht
- ST2B:     Ermittlung aberrometrischer Daten des zweiten Auges für die Nahsicht
- ST2C:     Ermittlung topographischer Daten des zweiten Auges
- ST1C:     Ermittlung topographischer Daten des ersten Auges

Soweit nur ein Auge vermessen werden soll, kann auf die Ermittlung der entsprechenden Daten des zweiten Auges verzichtet werden. In diesem Fall kann unmittelbar nach dem Schritt ST1B der Schritt ST1 C folgen. Dies ist in Fig. 2 durch einen gestrichelten Pfeil dargestellt.

Ein interessanter Aspekt ist die Erfassung von pupillometrischen Daten für zumindest zwei verschiedene Bedingungen der von dem jeweiligen Auge erfassten Helligkeit. Dabei ist es besonders bevorzugt, wenn die pupillometrischen Daten zusammen mit den aberrometrischen bzw. topographischen Daten ermittelt werden.

In einer bevorzugten Ausführungsform werden für die Erfassung der pupillometrischen Daten zwei verschiedene Helligkeitssollwerte festgelegt, welche insbesondere zwei tatsächlich zu erwartenden Helligkeitswerten einer individuellen Gebrauchssituation entsprechen. Damit werden vorzugsweise die Messkonditionen, unter denen die aberrometrischen Daten bzw. die topographischen Daten ermittelt werden, derart gewählt, dass das entsprechende Auge jeweils einer Helligkeit gemäß den festgelegten Helligkeitswerten ausgesetzt ist.

Dies ist allerdings nicht unbedingt notwendig. So können in einer anderen bevorzugten Ausführungsform die für die tatsächliche (individuelle) Gebrauchssituation erwarteten pupillometrischen Daten aus den unter anderen Messbedingungen erfassten pupillometrischen Daten abgeleitet werden. Dazu wird allerdings vorzugsweise die Helligkeit während der pupillometrischen Messung gemessen oder sie ist von vornherein bekannt. In einem vereinfachten Beispiel könnte die Größe (und Position) der Pupille für zwei verschiedenen Messkonditionen "sehr hell" und "sehr dunkel" erfasst werden, wohingegen die tatsächlich unter der Gebrauchssituation erwarteten Helligkeiten davon abweichende Werte "hell" und "dunkel" aufweisen. Die Größe (und Position) der Pupille für die Gebrauchssituation wird damit insbesondere durch (z.B. lineare oder logarithmische) Interpolation aus den gemessenen Größen berechnet. In analoger Weise kann auch eine Extrapolation genutzt werden.

Vorzugsweise werden die aberrometrischen Daten unter Messkonditionen mit geringer Helligkeit ermittelt. Dadurch erreicht man einen großen Pupillendurchmesser, wodurch wiederum eine hohe Genauigkeit der Messung erreicht wird. Vorzugsweise wird unter den Messkonditionen der aberrometrischen Messung, also bei geringer Helligkeit, die pupillometrische Messung für die geringere Helligkeit durchgeführt. Dies erfolgt besonders bevorzugt gleichzeitig zur aberrometrischen Messung oder die beiden Messungen erfolgen in unmittelbarer Folge aufeinander, d.h. ohne dass die Helligkeit in einer Weise verändert wird, die zu einer Veränderung der Pupille führen könnte.

Die topographischen Daten werden vorzugsweise unter Messkonditionen mit hoher Helligkeit (als sekundäre Helligkeit) ermittelt. Vorzugsweise wird dabei ein Lichtmuster (z.B. in Form von Placido-Ringen) auf die Hornhaut projiziert, dessen Reflexionen an der Hornhaut erfasst und zur Bestimmung der topographischen Daten ausgewertet werden. Durch die Verwendung von Messkonditionen mit hoher Helligkeit kann auch eine höhere Zuverlässigkeit und Genauigkeit der Auswertung der Reflexionen und damit eine höhere Präzision bei der Ermittlung der topographischen Daten erreicht werden. Vorzugsweise wird unter den Messkonditionen der topographischen Messung, also bei hoher Helligkeit, die pupillometrische Messung für die hohe Helligkeit durchgeführt (sekundäre pupillometrische Daten). Die erfolgt besonders bevorzugt gleichzeitig zur topographischen Messung oder die beiden Messungen erfolgen in unmittelbarer Folge aufeinander, d.h. ohne dass die Helligkeit in einer Weise verändert wird, die zu einer Veränderung der Pupille führen könnte.

Nachdem die Helligkeit des projizierten Lichtmusters sehr leicht verändert werden kann, ist ein bevorzugtes Messsystem vorzugsweise ausgelegt, die Helligkeit des Lichtmusters für die topographische Messung an die für die pupillometrische Messung gewünschte Helligkeit anzupassen. Vorzugsweise ist das Messsystem ausgelegt, eine für die Auswertung der Reflexionen genutzte Bilderfassungseinrichtung an die für die pupillometrische Messung gewünschte Helligkeit anzupassen.

Besonders bevorzugt werden die pupillometrischen Daten aus denselben Bilddaten erfasst wie die topographischen Daten. Es wird also vorzugsweise mittels einer Kamera nur ein Bild (Bilddatensatz) erzeugt, welches zur Auswertung sowohl der pupillometrischen Daten als auch der topographischen Daten herangezogen wird, was zu einer besonders schnellen Erfassung der Benutzerdaten mit geringem technischen Aufwand führt. Allerdings erfordert diese Vorgehensweise, dass die Pupillengrenzen in einem Bild ermittelt werden, in dem auch beispielsweise die Placido-Ringe sichtbar sind. Dies kann aber durch eine geeignete manuelle, semi-automatische oder automatische Vorgehensweise bewerkstelligt werden. Dabei kann insbesondere Zusatzinformation, wie z.B. eine Farbauflösung der Kamera oder eine zweite Kamera für die pupillometrische Messung mit einen (anderen) Farbfilter (als die für die topographische Messung genutzte erste Kamera) helfen, die relevanten Strukturen für die pupillometrische Messung und die topographische Messung im Bild bzw. in den Bildern zu unterscheiden. Die pupillometrischen und die topographischen Daten können aber auch aus einem gemeinsamen, monochromen Bild ermittelt werden, wenn man z.B. geometrische Randbedingungen für die jeweiligen Strukturen berücksichtigt.

Es ist nicht in allen Fällen erforderlich (oder gewünscht), topographische Daten zu erfassen. Selbst in einer bevorzugten Vorrichtung, welche insbesondere eine Topographie-Einheit mit einem Musterprojektor und einer Topographie-Auswerteeinrichtung umfasst, können die pupillometrischen Daten bei einem hohen Helligkeitswert auch erfasst werden, ohne dass topographische Daten erfasst werden. Dabei wird in einer bevorzugten Ausführungsform der Musterprojektor der Topographie-Einheit verwendet, um das zumindest eine Auge des Brillenträger bis zu einer gewünschten bzw. geeigneten hohen Helligkeit zu beleuchten, die für die Erfassung der pupillometrischen Daten bei hoher Helligkeit dient. In einer anderen bevorzugten Ausführungsform weist die Vorrichtung zusätzlich oder anstelle des Musterprojektors eine Beleuchtungseinrichtung (z.B. eine oder mehrere Lampen) auf, welche für die geforderte Helligkeit zur Erfassung der pupillometrischen Daten sorgt und besonders bevorzugt in der Helligkeit steuerbar ist, um beispielsweise die gemäß einer individuellen Gebrauchssituation erforderliche Helligkeit einstellen zu können.

Sofern die Vorrichtung eine Topographie-Einheit aufweist, ist es bevorzugt, wenn die Vorrichtung eine Abschattung des Auges gegen Umgebungslicht bewirkt, um in den Bilddaten einen möglichst guten Kontrast des projizierten Musters bzw. der Reflexionen auch in den Randbereichen der Hornhaut zu erreichen. Damit kann andererseits auch in einem hell beleuchteten Raum aufgrund der Abschattung des Auges eine pupillometrische Messung bei geringer Helligkeit durchgeführt werden.

Die Pupillengröße hängt nicht nur vom Beleuchtungszustand, sondern auch von der akkommodativen Stimulation ab. Es ist daher besonders bevorzugt, die Akkommodationsstimulanzeinrichtung (d.h. das virtuelle Target) auf die individuelle Gebrauchssituation einzustellen, für welche die pupillometrischen Daten erfasst werden sollen.

Die Integration von pupillometrischen Messungen in aberrometrischen und topographischen Messungen für Messbedingungen (insbesondere die Helligkeit und/oder den Akkommodationszustand), welche den individuellen Gebrauchssituationen entsprechen, oder die Ermittlung von Informationen für Bedingungen (insbesondere in Bezug auf Helligkeit und/oder Akkommodationszustand), welche nicht den gemessenen Bedingungen entsprechen, können in analoger Weise zu der oben für die Helligkeit beschriebenen Vorgehensweise (insbesondere durch Interpolation oder Extrapolation) erfolgen.

Während der Begriff "Fernsicht" den Eindruck erweckt, als beschreibe die Akkommodationsstimulanzeinrichtung eine weit entfernte aber endliche Position oder eine unendlich entfernte Position, ist es in der Praxis bevorzugt, dass für die "Fernsicht" das Auge in einen nicht akkommodieren Zustand (engl. "fogged state") versetzt wird. Dieser nicht akkommodierte Zustand eines Auges wird vorzugsweise dadurch erreicht, dass das virtuelle Target über eine unendlich entfernte Position hinaus (virtuell) bewegt wird. Das heißt, dass das optische System, das zur Projektion des Targets (z.B. ein Diapositiv) in das Auge verwendet wird, auf eine Brechkraft eingestellt wird, die ein wenig größer ist als die Refraktion des Auges. Damit kann der Proband nicht mehr auf das Target akkommodieren und das Auge erreicht einen entspannten, nicht akkommodierten Zustand.

Um verlässliche Werte bei der Bestimmung aberrometrischer Daten zu erreichen, ist es bevorzugt, dass die optische Brechkraft der Abbildungseinheit der Akkommodationsstimulanzeinrichtung größer ist als die Brechkraft des Auges als ein optisches System. Andernfalls könnte der Proband eine Akkommodation auf das Target erreichen. Das Resultat der aberrometrischen Messung könnte dann eher die Brechkraft der Abbildungseinheit widerspiegeln als die Brechkraft des entspannten Auges. Vorzugsweise wird ein genäherter Wert der Refraktion durch eine Vorabuntersuchung ermittelt. Diese Information wird dann für die virtuelle Positionierung des Targets herangezogen.

Im Kontext dieser Erfindung und der beschriebenen Beispiele umfasst der Begriff "Fernsicht" im Allgemeinen für eine Akkommodationsstimulanz entweder im entspannten Zustand ("fogged state") oder für eine unendliche Entfernung oder eine vorgegebene (große) Entfernung (z.B. etwa 50 m).

Vorzugsweise kann jeder Schritt des Ermittelns von aberrometrischen und/oder pupillometrischen und/oder topographischen Daten eine Vielzahl von Messungen unter denselben Messkonditionen umfassen. Diese Vielzahl von Messungen wird dann vorzugsweise durch eine statistische Auswertung kombiniert, um ein genaueres und verlässlicheres Resultat zu erhalten. Beispielsweise kann ein Mittelwert vieler Messungen gebildet werden, wobei Ausreisser in den Messungen außer Acht gelassen werden und/oder unplausible Messungen wiederholt werden. Dies gilt vorzugsweise nicht nur für die Fernsicht-Messungen, sondern alternativ oder zusätzlich auch für die pupillometrischen Messungen und/oder die topographischen Messungen und/oder die Nahsicht-Messungen.

Vorzugsweise weisen die Daten für die Nahsicht-Messung die analoge Struktur auf wie die Daten für die Fernsicht-Messung. Vorzugsweise besteht der einzige prinzipielle Unterschied in der Position des virtuellen Targets. Während für eine typische Fernsicht-Messung das Target auf eine virtuelle Position jenseits von unendlich bewegt wird, so dass das Auge nicht mehr akkommodieren kann, wird das Target für die Nahsicht-Messung auf eine virtuelle Position bewegt, die näher am Auge des Probanden liegt. Vorzugsweise wird die virtuelle Position ($D_p$) des Targets in Nahsicht aus der Brechkraft ($D_f$) in der Fernsicht, welche in der Fernsicht-Messung ermittelt wird, und der Brechkraft der Akkommodationsstimulanz ($D_a$) gemäß der Formel $D_p = D_f + D_a$ berechnet, wobei die Akkommodationsstimulanz üblicherweise negativ ist (z.B. -2,5 dpt). Um dem Probanden eine ausreichende Akkommodation für die Nahsicht erleichtern, ist es bevorzugt, das Target kontinuierlich und mit einer nicht zu großen Geschwindigkeit dem Auge zu nähern.

Für eine einzelne Nahsicht-Messung wird das Target vorzugsweise von einer Ausgangsposition (z.B. der Fernsicht-Position oder einer anderen Position, welche vorzugsweise weiter entfernt ist als die Nahsicht-Position) zur Nahsicht-Position $D_p$ bewegt und es werden nur Messungen bei $D_p$ durchgeführt. Für verfeinerte Untersuchungen können auch mehrere Nahsicht-Messungen nacheinander ausgeführt werden. Beispielsweise können zwei Nahsicht-Messungen durchgeführt werden: eine bei einer ersten Position $D_{p1}$ mit $D_{a1}$ = -1,0 dpt und eine weitere bei einer zweiten Position $D_{p2}$ mit $D_{a2}$ = -2,5 dpt. Vorzugsweise wird dazu das Target von einer Ausgangsposition zur ersten Position $D_{p1}$ bewegt, wo die erste Nahsicht-Messung durchgeführt wird. Vorzugsweise ohne eine weitere Verzögerung wird das Target weiterbewegt auf die zweite Position $D_{p2}$, wo die zweite Nahsicht-Messung durchgeführt wird. Natürlich muss der Proband dabei versuchen, der Akkommodationsstimulanz die ganze Zeit zu folgen.

Ungeachtet der Anzahl an Nahsicht-Messungen, die tatsächlich durchgeführt werden, ist es entscheidend, dass der Proband dem virtuellen Target durch die Akkommodation des Auges so gut wie möglich folgt. Andernfalls könnte das Auge in einem Zustand bleiben, der geringer akkommodiert oder gar entspannt ist, was die Messung verfälschen würde. Unterstützt wird die ständig ausreichende Akkommodation vorzugsweise dadurch, dass das Target anfangs bei einer virtuellen Position präsentiert wird, auf die der Proband leicht akkommodieren kann. Anschließend wird die virtuelle Position des Targets kontinuierlich mit nicht zu großer Geschwindigkeit verändert.

Vorzugsweise werden zusammen mit den pupillometrischen Daten für die hohe Helligkeit topographische Daten erfasst, da dies vorzugsweise ohne zusätzlichen Zeitaufwand bei der Messung möglich ist. Wie bereits ausgeführt, ist es allerdings nicht notwendig, dass topographische Daten erfasst werden, wenn beispielsweise das Auge der dafür notwendigen oder wünschenswerten Helligkeit nicht ausgesetzt werden soll oder wenn diese zusätzliche Information nicht erforderlich ist. In diesem Fall könnte auch auf die entsprechend hohe Empfindlichkeit der Bildaufnahmeeinrichtung und/oder den Musterprojektor sowie eine topographische Auswerteeinheit verzichtet werden.

Auch wenn eine topographische Messung durchgeführt wird, wird die Akkommodationsstimulanz vorzugsweise den Anforderungen der pupillometrischen Messung angepasst, da der Akkommodationszustand des Auges die Topographie der Hornhaut im Wesentlichen unbeeinflusst lässt bzw. dieser Einfluss vorzugsweise vernachlässt wird. Wie für die aberrometrischen Messungen, werden somit vorzugsweise auch für die pupillometrischen Messungen im hellen Zustand Messungen bei unterschiedlichen Akkommodationszuständen und/oder unterschiedlichen Helligkeiten durchgeführt. Wie bereits oben ausgeführt, werden die topographischen Daten vorzugsweise für die Optimierung der Refraktion und/oder des Glases auf Basis komplexerer Augenmodelle herangezogen.

Die gewählte Reihenfolge der Messungen bringt wesentliche Vorteile gegenüber anderen Vorgehensweisen mit sich. So wird beispielsweise dadurch, dass die Messungen bei höherer Helligkeit nach den Messungen bei geringerer Helligkeit durchgeführt werden die Genauigkeit insbesondere der aberrometrischen Messungen aber vorzugsweise auch der pupillometrischen Messungen verbessert, ohne den gesamten Zeitaufwand für die Datenerfassung wesentlich zu vergrößern. Dies lässt sich dadurch erklären, dass die Pupille einige Zeit benötigt, um sich an eine geringere Helligkeit zu gewöhnen, während die Verkleinerung der Pupille für die Anpassung an eine höhere Helligkeit deutlich schneller erfolgen kann.

Falls Messungen für beide Augen durchgeführt werden sollen, werden vorzugsweise die pupillometrischen Daten für den helleren Beleuchtungszustand beider Augen (und insbesondere auch die topographischen Messungen) erst nach den aberrometrischen Messungen beider Augen durchgeführt. Damit ein nachteiliger Einfluss auf die Messgenauigkeit aufgrund einer gegenseitigen Beeinflussung der beiden Augen reduziert. Insbesondere wurde festgestellt, dass der negative Einfluss auf die Messgenauigkeit aufgrund einer gegenseitigen akkommodativen Beeinflussung der beiden Augen weit geringer ist als ein negativer Einfluss auf die Messgenauigkeit aufgrund einer gegenseitigen adaptiven Beeinflussung durch die Helligkeit.

Außerdem wurde festgestellt, dass die Messgenauigkeit insbesondere aberrometrischen Messungen bis zu einem gewissen Maß mit einer Gewöhnung des Probanden bzw. des Auges an das Target und dessen virtuelle Bewegung verbessert wird. Es ist daher bevorzugt, zuerst beide bzw. sämtliche aberrometrischen Messungen des einen Auges durchzuführen, bevor das andere Auge gemessen wird. Außerdem wird dadurch der Aufwand für eine neue Justierung der Vorrichtung auf das Auge beim Augenwechsel reduziert. Aus diesem Grund wird auch für die pupillometrische Messung im helleren Zustand bzw. für die topographische Messung die Messreihenfolge der beiden Augen vorzugsweise vertauscht. Damit ist unmittelbar nach der letzten aberrometrischen Messung kein Augenwechsel und somit keine neue Justierung erforderlich.

In einer bevorzugten Ausführungsform umfasst das Verfahren zum Erfassen eines Satzes individueller Benutzerdaten für die Anpassung und Optimierung einer Brille vorzugsweise in folgenden Schritte 1 bis 9 in dieser Reihenfolge:

    1. Beginn mit dem ersten Auge, umfassend:

-    Vorrichtung wird auf das erste Auge justiert
-    Proband erfasst das Target mit dem ersten Auge

2. Aberrometrische Messung des ersten Auges in Fernsicht (erste aberrometrische Daten des ersten Auges), umfassend:

- Vorabmessung zur Bestimmung der erforderlichen virtuellen Distanz des Targets zur Stimulation des nicht akkommodierten Zustands ("fogged state")
- Pupillometrische Messung des ersten Auges für den dunkleren Zustand in Fernsicht (erste primäre pupillometrische Daten des ersten Auges)

3. Aberrometrische Messung des ersten Auges in Nahsicht (insbesondere basierend auf den Ergebnissen des vorangegangenen Schritts zur Ermittlung der virtuellen Position $D_a$ des Targets in der Nahsicht) (zweite aberrometrische Daten des ersten Auges), umfassend:

- Pupillometrische Messung des ersten Auges für den dunkleren Zustand in Nahsicht (zweite primäre pupillometrische Daten des ersten Auges)

4. Erster Augenwechsel, umfassend:

- Vorrichtung wird auf das zweite Auge justiert
- Proband erfasst das Target mit dem zweiten Auge

5. Aberrometrische Messung des ersten Auges in Fernsicht (erste aberrometrische Daten des zweiten Auges), umfassend:

- Vorabmessung zur Bestimmung der erforderlichen virtuellen Distanz des Targets zur Stimulation des nicht akkommodierten Zustands ("fogged state")
- Pupillometrische Messung des zweiten Auges für den dunkleren Zustand in Fernsicht (erste primäre pupillometrische Daten des zweiten Auges)

6. Aberrometrische Messung des zweiten Auges in Nahsicht (insbesondere basierend auf den Ergebnissen des vorangegangenen Schritts zur Ermittlung der virtuellen Position $D_a$ des Targets in der Nahsicht) (zweite aberrometrische Daten des zweiten Auges), umfassend:

- Pupillometrische Messung des zweiten Auges für den dunkleren Zustand in Nahsicht (zweite primäre pupillometrische Daten des zweiten Auges)

7. Topographische und pupillometrische Messung des zweiten Auges für den helleren Zustand (topographische Daten und sekundäre pupillometrische Daten des zweiten Auges)

8. Zweiter Augenwechsel, umfassend:

- Vorrichtung wird auf das erste Auges justiert
- Proband erfasst das Target mit dem ersten Auge

9. Topographische und pupillometrische Messung des ersten Auges für den helleren Zustand (topographische Daten und sekundäre pupillometrische Daten des ersten Auges)

Falls nur ein Auge vermessen werden soll, werden vorzugsweise alle Schritte für das zweite Auge weggelassen.

**Bezugszeichenliste**

| ST10 | Erfassen von ersten Messdaten |
| ST12 | Erfassen eines Serie von zweiten Messdaten |
| ST14 | Auswählen von zweiten Messdaten |
| ST16 | Erfassen eines photopischen Pupillenradius |
| ST18 | Skalieren der Zernike-Koeffizienten |
| ST20 | Ermittlung propagierter Refraktionswerte |
| ST22 | Ausgabe von Fernrefraktionswerten |

| | |
|---|---|
| ST24 | Transformation zu Power-Vektoren |
| ST26 | Ermittlung des Differenz-Power-Vektors |
| ST28 | Überprüfung der sphärischen Komponente des Differenz-Power-Vektors |
| ST30a, ST30b, ST30c | Ermittlung eines korrigierten Power-Vektors |
| ST 32 | Transformation des korrigierten Power-Vektors zu Refraktionsdaten |
| ST 34 | Ausgabe korrigierter Nah-Refraktionsdaten |
| ST1A | aberrometrische Messung des ersten Auges für die Fernsicht |
| ST1B | aberrometrische Messung des ersten Auges für die Nahsicht |
| ST1C | aberrometrische Messung des zweiten Auges für die Fernsicht |
| ST2A | aberrometrische Messung des zweiten Auges für die Nahsicht |
| ST2B | topographische Messung des zweiten Auges |
| ST2C | topographische Messung des ersten Auges |

**Patentansprüche**

1. Verfahren zur objektiven Refraktionsbestimmung für ein Auge eines Brillenträgers, umfassend ein Erfassen von Messdaten für das Auge des Brillenträgers, welche zumindest einen ersten Satz von Zernike-Koeffizienten $c_{2,F}^0$, $c_{2,F}^2$ und $c_{2,F}^{-2}$ zur Beschreibung einer Wellenfrontaberration bei einer Fernakkommodation des Auges und einem Pupillenradius $r_0$ einer Gebrauchssituation und einen zweiten Satz von Zernike-Koeffizienten $c_{2,N}^0$, $c_{2,N}^2$ und $c_{2,N}^{-2}$ zur Beschreibung einer Wellenfrontaberration bei einer Nahakkommodation des Auges und dem Pupillenradius $r_0$ einer Gebrauchssituation festlegen; wobei das Verfahren **dadurch gekennzeichnet ist, dass** das Verfahren ferner umfasst:

   - Ermitteln von objektiven Refraktionsdaten für Sphäre $Sph_N^{corr}$, Zylinder $Cyl_N^{corr}$ und Achslage $Axis_N^{corr}$ des Auges für einen Blick in die Nähe derart, dass die objektiven Refraktionsdaten in Abhängigkeit vom ersten und zweiten Satz von Zernike-Koeffizienten die Gleichungen

$$Sph_N^{corr} = M_N^{corr} + \sqrt{\left(J_{N,0}^{corr}\right)^2 + \left(J_{N,45}^{corr}\right)^2}$$

$$Cyl_N^{corr} = -2\sqrt{\left(J_{N,0}^{corr}\right)^2 + \left(J_{N,45}^{corr}\right)^2}$$

$$Axis_N^{corr} = \frac{1}{2}\arctan\left(-J_{N,0}^{corr}, -J_{N,45}^{corr}\right) + \frac{\pi}{2}$$

für einen korrigierten Power-Vektor $\mathbf{P}_N^{corr} = \begin{pmatrix} M_N^{corr} \\ J_{N,0}^{corr} \\ J_{N,45}^{corr} \end{pmatrix}$ für den Blick in die Nähe erfüllt, wobei der korrigierte

Power-Vektor $\mathbf{P}_N^{corr}$ in Abhängigkeit von einer Differenz

$$\Delta\mathbf{P} = \begin{pmatrix} \Delta M \\ \Delta \mathbf{J} \end{pmatrix} = \mathbf{P}_N - \mathbf{P}_F$$

zwischen einem ersten Power-Vektor

$$\mathbf{P}_F = \begin{pmatrix} M_F \\ \mathbf{J}_F \end{pmatrix} = \begin{pmatrix} -\dfrac{4\sqrt{3}}{r_0^2} c_{2,F}^0 \\ -\dfrac{2\sqrt{6}}{r_0^2} c_{2,F}^2 \\ -\dfrac{2\sqrt{6}}{r_0^2} c_{2,F}^{-2} \end{pmatrix}$$

und einem zweiten Power-Vektor

$$\mathbf{P}_N = \begin{pmatrix} M_N \\ \mathbf{J}_N \end{pmatrix} = \begin{pmatrix} -\dfrac{4\sqrt{3}}{r_0^2} c_{2,N}^0 \\ -\dfrac{2\sqrt{6}}{r_0^2} c_{2,N}^2 \\ -\dfrac{2\sqrt{6}}{r_0^2} c_{2,N}^{-2} \end{pmatrix}$$

-- dem Wert $\mathbf{P}_N - \begin{pmatrix} A_{1N} \\ 0 \\ 0 \end{pmatrix}$ entspricht, falls $\Delta M > A_{1N}$; und

-- dem Wert $\mathbf{P}_F + \dfrac{A_{1N}}{\Delta M} \begin{pmatrix} 0 \\ \Delta \mathbf{J} \end{pmatrix}$ entspricht, falls $\Delta M \le A_{1N}$, wobei $A_{1N} = -\dfrac{1}{d}$ das sphärische Äquivalent eines vorgegebenen Objektabstands $d$ für die Sicht in die Nähe ist, und wobei

$$\arctan(x, y) := \begin{cases} \arctan(y/x), & x > 0 \\ \arctan(y/x) + \pi, & x < 0, y > 0 \\ \arctan(y/x) - \pi, & x < 0, y < 0 \\ \pi/2, & x = 0, y > 0 \\ -\pi/2, & x = 0, y < 0 \end{cases}.$$

**2.** Verfahren nach Anspruch 1, wobei das Erfassen von Messdaten für das Auge des Brillenträgers umfasst:

- Erfassen von ersten Messdaten für das Auge des Brillenträgers für einen Blick in die Ferne, welche den ersten Satz von Zernike-Koeffizienten festlegen;
- Erfassen einer Serie von zweiten Messdaten für das Auge des Brillenträgers für einen Blick in die Nähe bei zumindest teilweise unterschiedlicher Akkommodation des Auges; und
- Auswählen derjenigen Messdaten aus der Serie, bei denen die stärkste Akkommodation des Auges auftritt, als zweite Messdaten, welche den zweiten Satz von Zernike-Koeffizienten festlegen.

**3.** Verfahren nach Anspruch 1 oder 2, wobei das Erfassen von Messdaten für das Auge des Brillenträgers umfasst:

- Erfassen von ersten Messdaten für das Auge des Brillenträgers für einen Blick in die Ferne, welche einen ersten Pupillenradius $R_F$ und einen ersten Satz von Zernike-Koeffizienten zur Beschreibung einer bei dem ersten Pupillenradius $R_F$ und der Fernakkommodation des Auges gemessenen Wellenfrontaberration umfassen;
- Erfassen von zweiten Messdaten für das Auge des Brillenträgers für einen Blick in die Nähe, welche einen zweiten Pupillenradius $R_N$ und einen zweiten Satz von Zernike-Koeffizienten zur Beschreibung einer bei dem zweiten Pupillenradius $R_N$ und der Nahakkommodation gemessenen Wellenfrontaberration umfassen; und
- Erfassen des Pupillenradius $r_0$ der Gebrauchssituation,
wobei der erste und der zweite Satz von Zernike-Koeffizienten zur Beschreibung der Wellenfrontaberrationen bei dem Pupillenradius $r_0$ der Gebrauchssituation durch Skalieren des ersten bzw. zweiten Satzes von Zernike-Koeffizienten zur Beschreibung der gemessenen Wellenfrontaberrationen in Abhängigkeit vom Verhältnis

$$\lambda_i = \frac{r_0}{R_i}$$ des Pupillenradius $r_0$ zum ersten ($i = F$) bzw. zweiten ($i = N$) Pupillenradius $R_i$ festgelegt ist.

4. Verfahren nach Anspruch 3, wobei der von den ersten bzw. zweiten Messdaten umfasste erste bzw. zweite Satz von Zernike-Koeffizienten zur Beschreibung der gemessenen Wellenfrontaberrationen zumindest Zernike-Koeffizienten bis zur vierten Ordnung umfasst und wobei der erste bzw. zweite Satz von Zernike-Koeffizienten zur Beschreibung der Wellenfrontaberrationen bei dem photopischen Pupillenradius $r_0$ gemäß

$$c_{2,i}^0(r_0) = \lambda_i^2 \left( c_{2,i}^0(R_i) + \sqrt{15}\left(\lambda_i^2 - 1\right)c_{4,i}^0(R_i) \right)$$

$$c_{2,i}^2(r_0) = \lambda_i^2 \left( c_2^2(R_i) + \sqrt{15}\left(\lambda_i^2 - 1\right)c_{4,i}^2(R_i) \right)$$

$$c_{2,i}^{-2}(r_0) = \lambda_i^2 \left( c_2^{-2}(R_i) + \sqrt{15}\left(\lambda_i^2 - 1\right)c_{4,i}^{-2}(R_i) \right)$$

vom ersten ($i = F$) bzw. zweiten ($i = N$) Satz von Zernike-Koeffizienten der gemessenen Wellenfrontaberration abhängt.

5. Verfahren nach Anspruch 3, wobei der von den ersten bzw. zweiten Messdaten umfasste erste bzw. zweite Satz von Zernike-Koeffizienten zur Beschreibung der gemessenen Wellenfrontaberrationen zumindest Zernike-Koeffizienten bis zur sechsten Ordnung umfasst und wobei der erste bzw. zweite Satz von Zernike-Koeffizienten zur Beschreibung der Wellenfrontaberrationen bei dem photopischen Pupillenradius $r_0$ gemäß

$$c_{2,i}^0(r_0) = \lambda_i^2 \left( c_{2,i}^0(R_i) + \sqrt{15}\left(\lambda_i^2 - 1\right)c_{4,i}^0(R_i) + \sqrt{21}\left(\lambda_i^2 - 1\right)\left(3\lambda_i^2 - 2\right)c_{6,i}^0(R_i) \right)$$

$$c_{2,i}^2(r_0) = \lambda_i^2 \left( c_{2,i}^2(R_i) + \sqrt{15}\left(\lambda_i^2 - 1\right)c_{4,i}^2(R_i) + \sqrt{21}\left(\lambda_i^2 - 1\right)\left(3\lambda_i^2 - 2\right)c_{6,i}^2(R_i) \right)$$

$$c_{2,i}^{-2}(r_0) = \lambda_i^2 \left( c_{2,i}^{-2}(R_i) + \sqrt{15}\left(\lambda_i^2 - 1\right)c_{4,i}^{-2}(R_i) + \sqrt{21}\left(\lambda_i^2 - 1\right)\left(3\lambda_i^2 - 2\right)c_{6,i}^{-2}(R_i) \right)$$

vom ersten ($i = F$) bzw. zweiten ($i = N$) Satz von Zernike-Koeffizienten der gemessenen Wellenfrontaberration abhängt.

6. Verfahren nach einem der vorangegangenen Ansprüche, wobei das Erfassen von Messdaten für das Auge des Brillenträgers umfasst:

- Erfassen von ersten Messdaten für das Auge des Brillenträgers für einen Blick in die Ferne, welche einen ersten Satz von Zernike-Koeffizienten zur Beschreibung einer bei der Fernakkommodation des Auges gemessenen Wellenfrontaberration an einer ersten Messposition umfassen; und
- Erfassen von zweiten Messdaten für das Auge des Brillenträgers für einen Blick in die Nähe, welche einen zweiten Satz von Zernike-Koeffizienten zur Beschreibung einer bei der Nahakkommodation des Auges gemessenen Wellenfrontaberration an einer zweiten Messposition umfassen; und wobei das Bestimmen von objektiven Refraktionsdaten des Auges für einen Blick in die Nähe umfasst:

- Ermitteln des ersten Power-Vektors $\mathbf{P}_F(VD_F) = \begin{pmatrix} M_F(VD_F) \\ J_F(VD_F) \end{pmatrix}$ aus dem von den ersten Messdaten umfassten oder gemäß einem der Ansprüche 3 bis 5 skalierten ersten Satz von Zernike-Koeffizienten in Abhängigkeit vom Abstand $VD_F$ einer Auswertungsposition von der ersten Messposition derart, dass $M_F(VD_F)$ das sphärische Äquivalentund $J_F(VD_F)$ den zylindrischen Anteil der Wellenfrontaberration an der Auswertungsposition bei der Fernakkommodation des Auges beschreiben; und

- Ermitteln des zweiten Power-Vektors $\mathbf{P}_N(VD_N) = \begin{pmatrix} M_N(VD_N) \\ J_N(VD_N) \end{pmatrix}$ aus dem von den zweiten Messdaten umfassten oder gemäß einem der Ansprüche 3 bis 5 skalierten zweiten Satz von Zernike-Koeffizienten in Abhängigkeit vom Abstand $VD_N$ der Auswertungsposition von der zweiten Messposition derart, dass $M_N(VD_N)$ das sphärische Äquivalent und $J_N(VD_N)$ den zylindrischen Anteil der Wellenfrontaberration an der Auswertungsposition bei der Nahakkommodation des Auges beschreiben.

7. Verfahren nach Anspruch 6, wobei der erste Power-Vektor und/oder der zweite Power-Vektor derart ermittelt wird, dass er die Gleichung

$$\mathbf{P}_i(VD_i) = \begin{pmatrix} Sph_i(VD_i) + \dfrac{Cyl_i(VD_i)}{2} \\ -\dfrac{Cyl_i(VD_i)}{2}\cos 2 Axis_i(VD_i) \\ -\dfrac{Cyl_i(VD_i)}{2}\sin 2 Axis_i(VD_i) \end{pmatrix}$$

mit

$$Sph_i(VD_i) = \frac{Sph_i(0)}{1 + VD_i \times Sph_i(0)}$$

$$Cyl_i(VD_i) = \frac{Sph_i(0) + Cyl_i(0)}{1 + VD_i \times (Sph_i(0) + Cyl_i(0))} - \frac{Sph_i(0)}{1 + VD_i \times Sph_i(0)}$$

$$Axis_i(VD_i) = Axis_i(0)$$

und

$$Sph_i(0) = -\frac{4\sqrt{3}}{r_0^2}c_{2,i}^0 + \frac{2\sqrt{6}}{r_0^2}\sqrt{\left(c_{2,i}^{-2}\right)^2 + \left(c_{2,i}^2\right)^2}$$

$$Cyl_i(0) = -\frac{4\sqrt{6}}{r_0^2}\sqrt{\left(c_{2,i}^{-2}\right)^2 + \left(c_{2,i}^2\right)^2}$$

$$Axis_i(0) = \frac{1}{2}\arctan\left(c_{2,i}^2, c_{2,i}^{-2}\right) + \frac{\pi}{2}$$

für $i = F, N$ erfüllt, wobei

$$\arctan(x, y) := \begin{cases} \arctan(y/x), & x > 0 \\ \arctan(y/x) + \pi, & x < 0, y > 0 \\ \arctan(y/x) - \pi, & x < 0, y < 0 \\ \pi/2, & x = 0, y > 0 \\ -\pi/2, & x = 0, y < 0 \end{cases}.$$

und wobei $c_{2,i}^0$, $c_{2,i}^2$ und $c_{2,i}^{-2}$ die von den ersten ($i = F$) bzw. zweiten ($i = N$) Messdaten umfassten bzw. gemäß einem der Ansprüche 3 bis 5 skalierten Zernike-Koeffizienten zweiter Ordnung für den Blick in die Ferne ($i = F$) bzw. den Blick in die Nähe ($i = N$) bezeichnen.

8. Verfahren nach einem der vorangegangenen Ansprüche, wobei das Erfassen der ersten und/oder zweiten Messdaten ein Messen von Refraktionsdaten des Auges mittels eines Autorefraktors und/oder mittels eines Aberrometers umfasst.

9. Vorrichtung zur objektiven Refraktionsbestimmung für ein Auge eines Brillenträgers, umfassend eine Messdatenerfassungsschnittstelle zum Erfassen von Messdaten für das Auge des Brillenträgers, welche zumindest einen ersten Satz von Zernike-Koeffizienten $c_{2,F}^0$, $c_{2,F}^2$ und $c_{2,F}^{-2}$ zur Beschreibung einer Wellenfrontaberration bei einer Fernakkommodation und einem photopischen Pupillenradius $r_0$ des Auges und einen zweiten Satz von Zernike-Koeffizienten $c_{2,N}^0$, $c_{2,N}^2$ und $c_{2,N}^{-2}$ zur Beschreibung einer Wellenfrontaberration bei einer Nahakkommodation und dem photopischen Pupillenradius $r_0$ des Auges festlegen;
wobei die Vorrichtung **dadurch gekennzeichnet ist, dass** die Vorrichtung ferner umfasst:

- eine Refraktionsdatenermittlungseinrichtung zum Ermitteln von objektiven Refraktionsdaten für Sphäre $\left(Sph_N^{corr}\right)$, Zylinder $\left(Cyl_N^{corr}\right)$ und Achslage $\left(Axis_N^{corr}\right)$ des Auges für einen Blick in die Nähe derart, dass die objektiven Refraktionsdaten in Abhängigkeit vom ersten und zweiten Satz von Zernike-Koeffizienten die Gleichungen

$$Sph_N^{corr} = M_N^{corr} + \sqrt{\left(J_{N,0}^{corr}\right)^2 + \left(J_{N,45}^{corr}\right)^2}$$

$$Cyl_N^{corr} = -2\sqrt{\left(J_{N,0}^{corr}\right)^2 + \left(J_{N,45}^{corr}\right)^2}$$

$$Axis_N^{corr} = \frac{1}{2}\arctan\left(-J_{N,0}^{corr}, -J_{N,45}^{corr}\right) + \frac{\pi}{2}$$

für einen korrigierten Power-Vektor $\mathbf{P}_N^{corr} = \begin{pmatrix} M_N^{corr} \\ J_{N,0}^{corr} \\ J_{N,45}^{corr} \end{pmatrix}$ für den Blick in die Nähe erfüllt, wobei der korrigierte

Power-Vektor $\mathbf{P}_N^{corr}$ in Abhängigkeit von einer Differenz $\Delta\mathbf{P} = \begin{pmatrix} \Delta M \\ \Delta\mathbf{J} \end{pmatrix} = \mathbf{P}_N - \mathbf{P}_F$ zwischen einem ersten

Power-Vektor

$$\mathbf{P}_F = \begin{pmatrix} M_F \\ \mathbf{J}_F \end{pmatrix} = \begin{pmatrix} -\dfrac{4\sqrt{3}}{r_0^2}c_{2,F}^0 \\ -\dfrac{2\sqrt{6}}{r_0^2}c_{2,F}^2 \\ -\dfrac{2\sqrt{6}}{r_0^2}c_{2,F}^{-2} \end{pmatrix}$$

und einem zweiten Power-Vektor

$$\mathbf{P}_N = \begin{pmatrix} M_N \\ \mathbf{J}_N \end{pmatrix} = \begin{pmatrix} -\dfrac{4\sqrt{3}}{r_0^2}c_{2,N}^0 \\ -\dfrac{2\sqrt{6}}{r_0^2}c_{2,N}^2 \\ -\dfrac{2\sqrt{6}}{r_0^2}c_{2,N}^{-2} \end{pmatrix}$$

-- dem Wert $\mathbf{P}_N - \begin{pmatrix} A_{1N} \\ 0 \\ 0 \end{pmatrix}$ entspricht, falls $\Delta M > A_{1N}$; und

-- dem Wert $\mathbf{P}_F + \dfrac{A_{1N}}{\Delta M}\begin{pmatrix} 0 \\ \Delta\mathbf{J} \end{pmatrix}$ entspricht, falls $\Delta M \leq A_{1N}$, wobei $A_{1N} = -\dfrac{1}{d}$ das sphärische Äquivalent eines vorgegebenen Objektabstands $d$ für die Sicht in die Nähe ist, und wobei

$$\arctan(x, y) := \begin{cases} \arctan(y/x), & x > 0 \\ \arctan(y/x) + \pi, & x < 0, y > 0 \\ \arctan(y/x) - \pi, & x < 0, y < 0 \\ \pi/2, & x = 0, y > 0 \\ -\pi/2, & x = 0, y < 0 \end{cases}.$$

**10.** Computerprogrammprodukt umfassend computerlesbare Anweisungen, welche, wenn geladen in einen Speicher eines Computers und ausgeführt von dem Computer, bewirken, dass der Computer ein Verfahren gemäß einem der Ansprüche 1 bis 8 durchführt.

**Claims**

**1.** Method for determining objective refraction for an eye of a spectacle wearer comprising acquiring measurement data for the eye of the spectacle wearer, which establish at least a first set of Zernike coefficients $c_{2,F}^0$, $c_{2,F}^2$ and

$c_{2,F}^{-2}$ for representation of a wavefront aberration in the case of a far point accommodation of the eye and a pupil radius $r_0$ of a situation of use and a second set of Zernike coefficients $c_{2,N}^0$, $c_{2,N}^2$ and $c_{2,N}^{-2}$ for representation of a wavefront aberration in the case of a near point accommodation of the eye and a pupil radius $r_0$ of a situation of use, wherein the method is **characterised in that** the method further comprises:

- determining objective refraction data for sphere $Sph_N^{corr}$, cylinder $Cyl_N^{corr}$ and axis position $Axis_N^{corr}$ of the eye for a near view in such a way that as a function of the first and second set of Zernike coefficients the objective refraction data fulfil the equations

$$Sph_N^{corr} = M_N^{corr} + \sqrt{(J_{N,0}^{corr})^2 + (J_{N,45}^{corr})^2}$$

$$Cyl_N^{corr} = -2\sqrt{(J_{N,0}^{corr})^2 + (J_{N,45}^{corr})^2}$$

$$Axis_N^{corr} = \frac{1}{2}arctan(-J_{N,0}^{corr}, -J_{N,45}^{corr}) + \frac{\pi}{2}$$

for a corrected power vector $P_N^{corr} = \begin{pmatrix} M_N^{corr} \\ J_{N,0}^{corr} \\ J_{N,45}^{corr} \end{pmatrix}$ for the near view,

wherein as a function of a difference $\Delta P = \begin{pmatrix} \Delta M \\ \Delta J \end{pmatrix} = P_N - P_F$ between a first power vector

$$P_F = \begin{pmatrix} M_F \\ J_F \end{pmatrix} = \begin{pmatrix} -\frac{4\sqrt{3}}{r_0^2}c_{2,F}^0 \\ -\frac{2\sqrt{6}}{r_0^2}c_{2,F}^2 \\ -\frac{2\sqrt{6}}{r_0^2}c_{2,F}^{-2} \end{pmatrix}$$ and a second power vector

$$P_N = \begin{pmatrix} M_N \\ J_N \end{pmatrix} = \begin{pmatrix} -\frac{4\sqrt{3}}{r_0^2}c_{2,N}^0 \\ -\frac{2\sqrt{6}}{r_0^2}c_{2,N}^2 \\ -\frac{2\sqrt{6}}{r_0^2}c_{2,N}^{-2} \end{pmatrix}$$

the corrected power vector $P_N^{corr}$ corresponds to

- the value $P_N - \begin{pmatrix} A_{1N} \\ 0 \\ 0 \end{pmatrix}$ if $\Delta M > A_{1N}$, and to wherein $A_{1N} = -\frac{1}{d}$ is the spherical equivalent of a predetermined object distance $d$ for the near vision, and wherein

$$\arctan(x,y) = \begin{pmatrix} \arctan(y\,/\,x)\,, x > 0 \\ arctan(y/x) + \pi, x < 0, y > 0 \\ arctan(y/x) - \pi, x < 0, y < 0 \\ \pi/2\,, x = 0, y > 0 \\ -\pi/2\,, x = 0, y < 0 \end{pmatrix}$$

**2.** Method according to claim 1, wherein the acquisition of measurement data for the eye of the spectacle wearer comprises:

- acquiring first measurement data for the eye of the spectacle wearer for a distant view, which establish the first set of Zernike coefficients;
- acquiring a series of second measurement data for the eye of the spectacle wearer for a near view in the case of at least partially different accommodation of the eye; and
- selecting those measurement data of the series with which the strongest accommodation of the eye occurs as second measurement data, which establish the second set of Zernike coefficients.

**3.** Method according to claim 1 or 2, wherein the acquisition of measurement data for the eye of the spectacle wearer comprises:

- acquiring first measurement data for the eye of the spectacle wearer for a distant view, which comprise a first pupil radius $R_F$ and a first set of Zernike coefficients for representation of a wavefront aberration measured in the case of the first pupil radius $R_F$ and the far point accommodation of the eye;
- acquiring second measurement data for the eye of the spectacle wearer for a near view, which comprise a second pupil radius $R_N$ and a second set of Zernike coefficients for representation of a wavefront aberration measured in the case of the second pupil radius $R_N$ and the near point accommodation of the eye; and
- acquiring the pupil radius $r_0$ of the situation of use,

wherein the first and the second set of Zernike coefficients for representation of the wavefront aberrations in the case of the pupil radius $r_0$ of the situation of use are established by scaling the first or second set of Zernike coefficients for representation of the measured wavefront aberrations as a function of the relation $\lambda_i = \dfrac{r_0}{R_i}$ of the pupil radius *roto* the first (*i=F*) or second (*i=N*) pupil radius $R_i$.

**4.** Method according to claim 3, wherein the first or second set of Zernike coefficients comprised by the first or second measurement data for representation of the measured wavefront aberrations comprises at least Zernike coefficients up to the fourth order and wherein the first or second set of Zernike coefficients for representation of the wavefront aberrations in the case of the photopic pupil radius $r_0$ according to

$$c_{2,i}^0(r_0) = \lambda_i^2\left(c_{2,i}^0(R_i) + \sqrt{15}(\lambda_i^2 - 1)c_{4,i}^0(R_i)\right)$$

$$c_{2,i}^2(r_0) = \lambda_i^2(c_2^2(R_i) + \sqrt{15}(\lambda_i^2 - 1)c_{4,i}^2(R_i))$$

$$c_{2,i}^{-2}(r_0) = \lambda_i^2(c_2^{-2}(R_i) + \sqrt{15}(\lambda_i^2 - 1)c_{4,i}^{-2}(R_i))$$

is dependent on the first (*i=F*) or second (*i=N*) set of Zernike coefficients of the measured wavefront aberration.

**5.** Method according to claim 3, wherein the first or second set of Zernike coefficients comprised by the first or second measurement data for representation of the measured wavefront aberrations comprises at least Zernike coefficients up to the sixth order and wherein the first or second set of Zernike coefficients for representation of the wavefront aberrations in the case of the photopic pupil radius $r_0$ according to

$$c_{2,i}^0(r_0) = \lambda_i^2\left(c_{2,i}^0(R_i) + \sqrt{15}(\lambda_i^2 - 1)c_{4,i}^0(R_i) + \sqrt{21}(\lambda_i^2 - 1)(3\lambda_i^2 - 2)c_{6,i}^0(R_i)\right)$$

$$c_{2,i}^2(r_0) = \lambda_i^2\left(c_2^2(R_i) + \sqrt{15}(\lambda_i^2 - 1)c_{4,i}^2(R_i) + \sqrt{21}(\lambda_i^2 - 1)(3\lambda_i^2 - 2)c_{6,i}^2(R_i)\right)$$

$$c_{2,i}^{-2}(r_0) = \lambda_i^2\left(c_2^{-2}(R_i) + \sqrt{15}(\lambda_i^2 - 1)c_{4,i}^{-2}(R_i) + \sqrt{21}(\lambda_i^2 - 1)(3\lambda_i^2 - 2)c_{6,i}^{-2}(R_i)\right)$$

is dependent on the first ($i=F$) or second ($i=N$) set of Zernike coefficients of the measured wavefront aberration.

6. Method according to one of the preceding claims, wherein the acquisition of measurement data for the eye of the spectacle wearer comprises:

- acquiring first measurement data for the eye of the spectacle wearer for a distant view, which comprise a first set of Zernike coefficients for representation of a wavefront aberration measured in the case of the far point accommodation of the eye at a first measurement position; and
- acquiring second measurement data for the eye of the spectacle wearer for a near view, which comprise a second set of Zernike coefficients for representation of a wavefront aberration measured in the case of the near point accommodation of the eye at a second measurement position;

and wherein the determination of objective refraction data of the eye for a near view comprises:

- determining the first power vector $P_F(VD_F) = \begin{pmatrix} M_F(VD_F) \\ J_F(VD_F) \end{pmatrix}$ from the first set of Zernike coefficients comprised in the first measurement data or scaled according to one of claims 3 to 5 as a function of distance $VD_F$ of an evaluation position from the first measurement position in such a way that $M_F(VD_F)$ denotes the spherical equivalent and $J_F(VD_F)$ denotes the cylindrical component of the wavefront aberration at the evaluation position in the case of the far point accommodation of the eye; and

- determining the second power vector $P_N(VD_N) = \begin{pmatrix} M_N(VD_N) \\ J_N(VD_N) \end{pmatrix}$ from the second set of Zernike coefficients comprised in the first measurement data or scaled according to one of claims 3 to 5 as a function of distance $VD_N$ of the evaluation position from the first measurement position in such a way that $M_N(VD_N)$ denotes the spherical equivalent and $J_N(VD_N)$ denotes the cylindrical component of the wavefront aberration at the evaluation position in the case of the near point accommodation of the eye.

7. Method according to claim 6, wherein the first power vector and/or the second power vector is determined in such a way that it fulfils the equation

$$P_i(VD_i) = \begin{pmatrix} Sph_i(VD_i) + \dfrac{Cyl_i(VD_i)}{2} \\ -\dfrac{Cyl_i(VD_i)}{2}\cos 2Axis_i(VD_i) \\ -\dfrac{Cyl_i(VD_i)}{2}\cos 2Axis_i(VD_i) \end{pmatrix}$$

with

$$Sph_i(VD_i) = \frac{Sph_i(0)}{1 + VD_i \times Sph_i(0)}$$

$$Cyl_i(VD_i) = \frac{Sph_i(0) + Cyl_i(0)}{1 + VD_i \times (Sph_i(0) + Cyl_i(0))} - \frac{Sph_i(0)}{1 + VD_i \times Sph_i(0)}$$

$$Axis_i(VD_i) = Axis_i(0)$$

and

$$Sph_i(0) = -\frac{4\sqrt{3}}{r_0^2}c_{2,i}^0 + \frac{2\sqrt{6}}{r_0^2}\sqrt{(c_{2,i}^{-2})^2 + (c_{2,i}^2)^2}$$

$$Cyl_i(0) = -\frac{4\sqrt{6}}{r_0^2}\sqrt{(c_{2,i}^{-2})^2 + (c_{2,i}^2)^2}$$

$$Axis_i(0) = \frac{1}{2}arctan(c_{2,i}^2, c_{2,i}^{-2}) + \frac{\pi}{2}$$

for *i = F, N,* wherein

$$arctan(x,y) = \begin{pmatrix} arctan(y/x), x > 0 \\ arctan(y/x) + \pi, x < 0, y > 0 \\ arctan(y/x) - \pi, x < 0, y < 0 \\ \pi/2, x = 0, y > 0 \\ -\pi/2, x = 0, y < 0 \end{pmatrix}$$

and wherein $c_{2,i}^0, c_{2,i}^2$ and $c_{2,i}^{-2}$ represent the Zernike coefficients of the second order comprised in the first (*i = F*) or second (*i = N*) measurement data or scaled according to one of claims 3 to 5 for the distant view *(i = F)* or the near view *(i = N).*

8. Method according to one of the preceding claims, wherein the acquisition of the first and/or second measurement data comprises a measurement of refraction data of the eye by means of an autorefractor and/or by means of an aberrometer.

9. Device for determining the objective refraction for an eye of a spectacle wearer comprising a measurement data interface for acquiring measurement data for the eye of the spectacle wearer, which establish at least a first set of Zernike coefficients $c_{2,F}^0$, $c_{2,F}^2$ and $c_{2,F}^{-2}$ for representation of a wavefront aberration in the case of a far point accommodation and a photopic pupil radius $r_0$ of the eye and a second set of Zernike coefficients $c_{2,N}^0$, $c_{2,N}^2$ and $c_{2,N}^{-2}$ for representation of a wavefront aberration in the case of a near point accommodation and the photopic pupil radius $r_0$ of the eye,

wherein the device is **characterised in that** the device further comprises:

   - a refraction data determination device for determining objective refraction data for sphere $(Sph_N^{corr})$, cylinder $(Cyl_N^{corr})$ and axis position $(Axis_N^{corr})$ of the eye for a near view in such a way that as a function of the first and second set of Zernike coefficients the objective refraction data fulfil the equations

$$Sph_N^{corr} = M_N^{corr} + \sqrt{(J_{N,0}^{corr})^2 + (J_{N,45}^{corr})^2}$$

$$Cyl_N^{corr} = -2\sqrt{(J_{N,0}^{corr})^2 + (J_{N,45}^{corr})^2}$$

$$Axis_N^{corr} = \frac{1}{2} arctan\left(-J_{N,0}^{corr}, -J_{N,45}^{corr}\right) + \frac{\pi}{2}$$

for a corrected power vector $P_N^{corr} = \begin{pmatrix} M_N^{corr} \\ J_{N,0}^{corr} \\ J_{N,45}^{corr} \end{pmatrix}$ for the near view, wherein as a function of a difference

$$\Delta P = \begin{pmatrix} \Delta M \\ \Delta J \end{pmatrix} = P_N - P_F \text{ between a first power vector } P_F = \begin{pmatrix} M_F \\ J_F \end{pmatrix} = \begin{pmatrix} -\frac{4\sqrt{3}}{r_0^2} c_{2,F}^0 \\ -\frac{2\sqrt{6}}{r_0^2} c_{2,F}^2 \\ -\frac{2\sqrt{6}}{r_0^2} c_{2,F}^{-2} \end{pmatrix} \text{ and a second}$$

power vector

$$P_N = \begin{pmatrix} M_N \\ J_N \end{pmatrix} = \begin{pmatrix} -\frac{4\sqrt{3}}{r_0^2} c_{2,N}^0 \\ -\frac{2\sqrt{6}}{r_0^2} c_{2,N}^2 \\ -\frac{2\sqrt{6}}{r_0^2} c_{2,N}^{-2} \end{pmatrix}$$

the corrected power vector $P_N^{corr}$ corresponds to

- the value $P_N - \begin{pmatrix} A_{1N} \\ 0 \\ 0 \end{pmatrix}$ if $\Delta M > A_{1N}$, and to

- the value $P_F + \frac{A_{1N}}{\Delta M}\begin{pmatrix} 0 \\ \Delta J \end{pmatrix}$ if $\Delta M \le A_{1N}$, wherein $A_{1N} = -\frac{1}{d}$ is the spherical equivalent of a predetermined object distance $d$ for the near vision, and wherein

$$arctan(x, y) = \begin{cases} arctan(y/x), x > 0 \\ arctan(y/x) + \pi, x < 0, y > 0 \\ arctan(y/x) - \pi, x < 0, y < 0 \\ \pi/2, x = 0, y > 0 \\ -\pi/2, x = 0, y < 0 \end{cases}$$

**10.** Computer program product comprising computer-readable instructions, which when loaded into a memory of a computer and implemented by the computer cause the computer to perform a method according to one of claims 1 to 8.

**Revendications**

**1.** Procédé de détermination de réfraction objective pour un oeil d'un porteur de lunettes, comprenant une saisie de données de mesure pour l'oeil du porteur de lunettes, lesquelles définissent au moins un premier ensemble de polynômes de Zernike $c_{2,F}^0$, $c_{2,F}^2$ et $c_{2,F}^{-2}$ pour la description d'une aberration de front d'onde avec une accommodation de l'oeil en vision de loin et un rayon de pupille $r_0$ d'une situation d'utilisation, et un deuxième ensemble de polynômes de Zernike $c_{2,N}^0$, $c_{2,N}^2$ et $c_{2,N}^{-2}$ pour la description d'une aberration de front d'onde avec une accommodation de l'oeil en vision de près, et du rayon de pupille $r_0$ d'une situation d'utilisation ; ledit procédé étant **caractérisé en ce qu'**il comprend en outre :

- la détermination de données de réfraction objectives pour la sphère $Sph_N^{corr}$, le cylindre $Cyl_N^{corr}$ et la position d'axe $Axis_N^{corr}$ de l'oeil pour une vision de près, de sorte qu'en fonction du premier et du deuxième ensemble de polynômes de Zernike, les données de réfraction objectives satisfassent aux égalités

$$Sph_N^{corr} = M_N^{corr} + \sqrt{\left(J_{N,0}^{corr}\right)^2 + \left(J_{N,45}^{corr}\right)^2}$$

$$Cyl_N^{corr} = -2\sqrt{\left(J_{N,0}^{corr}\right)^2 + \left(J_{N,45}^{corr}\right)^2}$$

$$Axis_N^{corr} = \frac{1}{2}\arctan\left(-J_{N,0}^{corr}, -J_{N,45}^{corr}\right) + \frac{\pi}{2}$$

pour un vecteur de puissance corrigé $\mathbf{P}_N^{corr} = \begin{pmatrix} M_N^{corr} \\ J_{N,0}^{corr} \\ J_{N,45}^{corr} \end{pmatrix}$ pour la vision de près, où, en fonction d'une différence

$$\Delta\mathbf{P} = \begin{pmatrix} \Delta M \\ \Delta \mathbf{J} \end{pmatrix} = \mathbf{P}_N - \mathbf{P}_F$$ entre un premier vecteur de puissance $\mathbf{P}_F = \begin{pmatrix} M_F \\ \mathbf{J}_F \end{pmatrix} = \begin{pmatrix} -\frac{4\sqrt{3}}{r_0^2}c_{2,F}^0 \\ -\frac{2\sqrt{6}}{r_0^2}c_{2,F}^2 \\ -\frac{2\sqrt{6}}{r_0^2}c_{2,F}^{-2} \end{pmatrix}$ et un deuxième vecteur

de puissance

$$\mathbf{P}_N = \begin{pmatrix} M_N \\ \mathbf{J}_N \end{pmatrix} = \begin{pmatrix} -\frac{4\sqrt{3}}{r_0^2}c_{2,N}^0 \\ -\frac{2\sqrt{6}}{r_0^2}c_{2,N}^2 \\ -\frac{2\sqrt{6}}{r_0^2}c_{2,N}^{-2} \end{pmatrix},$$

le vecteur de puissance $P_N^{corr}$

- correspond à la valeur $\mathbf{P}_N - \begin{pmatrix} A_{1N} \\ 0 \\ 0 \end{pmatrix}$, si $\Delta M > A_{1N}$ ; et

- correspond à la valeur $\mathbf{P}_F + \frac{A_{1N}}{\Delta M}\begin{pmatrix} 0 \\ \Delta \mathbf{J} \end{pmatrix}$, si $\Delta M \leq A_{1N}$, où $A_{1N} = -\frac{1}{d}$ est l'équivalent sphérique d'une distance d'objet définie $d$ pour la vision de près, et où

$$\arctan(x, y) := \begin{cases} \arctan(y/x), & x > 0 \\ \arctan(y/x) + \pi, & x < 0, y > 0 \\ \arctan(y/x) - \pi, & x < 0, y < 0 \\ \pi/2, & x = 0, y > 0 \\ -\pi/2, & x = 0, y < 0 \end{cases}.$$

**2.** Procédé selon la revendication 1, où la saisie de données de mesure pour l'oeil du porteur de lunettes comprend :

- la saisie de premières données de mesure pour l'oeil du porteur de lunettes pour une vision de loin, lesquelles définissent le premier ensemble de polynômes de Zernike ;
- la saisie d'une série de deuxièmes données de mesure pour l'oeil du porteur de lunettes pour une vision de près avec une accommodation de l'oeil au moins partiellement différente ; et
- la sélection des données de mesure dans la série où est présentée la plus forte accommodation de l'oeil, en tant que deuxièmes données de mesure définissant le deuxième ensemble de polynômes de Zernike.

**3.** Procédé selon la revendication 1 ou 2, où la saisie de données de mesure pour l'oeil du porteur de lunettes comprend :

- la saisie de premières données de mesure pour l'oeil du porteur de lunettes pour une vision de loin, lesquelles comprennent un premier rayon de pupille $R_F$ et un premier ensemble de polynômes de Zernike pour la description d'une aberration de front d'onde mesurée avec le premier rayon de pupille $R_F$ et l'accommodation de l'oeil en vision de loin ;
- la saisie de deuxièmes données de mesure pour l'oeil du porteur de lunettes pour une vision de près, lesquelles comprennent un deuxième rayon de pupille $R_N$ et un deuxième ensemble de polynômes de Zernike pour la description d'une aberration de front d'onde mesurée avec le deuxième rayon de pupille $R_N$ et l'accommodation en vision de près ; et
- la saisie du rayon de pupille $r_0$ de la situation d'utilisation,

le premier et le deuxième ensembles de polynômes de Zernike pour la description des aberrations de front d'onde avec le rayon de pupille $r_0$ de la situation d'utilisation étant définis par ajustement du premier ou du deuxième ensemble de polynômes de Zernike pour la description des aberrations de front d'onde mesurées en fonction du rapport $\lambda_i = \dfrac{r_0}{R_i}$ entre le rayon de pupille $r_0$ et le premier ($i = F$) ou le deuxième (i = N) rayon de pupille $R_i$.

**4.** Procédé selon la revendication 3, où le premier ou le deuxième ensemble de polynômes de Zernike pour la description des aberrations de front d'onde mesurées, compris dans les premières ou les deuxièmes données de mesure, comprend au moins des polynômes de Zernike jusqu'au quatrième ordre, et où le premier ou le deuxième ensemble de polynômes de Zernike pour la description des aberrations de front d'onde avec le rayon de pupille photopique $r_0$ suivant

$$c_{2,i}^0(r_0) = \lambda_i^2\left(c_{2,i}^0(R_i) + \sqrt{15}\left(\lambda_i^2 - 1\right)c_{4,i}^0(R_i)\right)$$

$$c_{2,i}^2(r_0) = \lambda_i^2\left(c_2^2(R_i) + \sqrt{15}\left(\lambda_i^2 - 1\right)c_{4,i}^2(R_i)\right)$$

$$c_{2,i}^{-2}(r_0) = \lambda_i^2\left(c_2^{-2}(R_i) + \sqrt{15}\left(\lambda_i^2 - 1\right)c_{4,i}^{-2}(R_i)\right)$$

est fonction du premier ($i = F$) ou du deuxième ($i = N$) ensemble de polynômes de Zernike de l'aberration de front d'onde mesurée.

**5.** Procédé selon la revendication 3, où le premier ou le deuxième ensemble de polynômes de Zernike pour la description des aberrations de front d'onde mesurées, compris dans les premières ou les deuxièmes données de mesure, comprend au moins des polynômes de Zernike jusqu'au sixième ordre, et où le premier ou le deuxième ensemble de polynômes de Zernike pour la description des aberrations de front d'onde avec le rayon de pupille photopique $r_0$ suivant

$$c_{2,i}^0(r_0) = \lambda_i^2\left(c_{2,i}^0(R_i) + \sqrt{15}\left(\lambda_i^2 - 1\right)c_{4,i}^0(R_i) + \sqrt{21}\left(\lambda_i^2 - 1\right)\left(3\lambda_i^2 - 2\right)c_{6,i}^0(R_i)\right)$$

$$c_{2,i}^{2}(r_0) = \lambda_i^2\left(c_{2,i}^{2}(R_i) + \sqrt{15}\left(\lambda_i^2 - 1\right)c_{4,i}^{2}(R_i) + \sqrt{21}\left(\lambda_i^2 - 1\right)\left(3\lambda_i^2 - 2\right)c_{6,i}^{2}(R_i)\right)$$

$$c_{2,i}^{-2}(r_0) = \lambda_i^2\left(c_{2,i}^{-2}(R_i) + \sqrt{15}\left(\lambda_i^2 - 1\right)c_{4,i}^{-2}(R_i) + \sqrt{21}\left(\lambda_i^2 - 1\right)\left(3\lambda_i^2 - 2\right)c_{6,i}^{-2}(R_i)\right)$$

est fonction du premier (*i = F*) ou du deuxième (*i = N*) ensemble de polynômes de Zernike de l'aberration de front d'onde mesurée.

6. Procédé selon l'une des revendications précédentes, où la saisie de données de mesure pour l'oeil du porteur de lunettes comprend :

   - la saisie de premières données de mesure pour l'oeil du porteur de lunettes pour une vision de loin, lesquelles comprennent un premier ensemble de polynômes de Zernike pour la description d'une aberration de front d'onde mesurée avec l'accommodation de l'oeil en vision de loin sur une première position de mesure ; et
   - la saisie de deuxièmes données de mesure pour l'oeil du porteur de lunettes pour une vision de près, lesquelles comprennent un deuxième ensemble de polynômes de Zernike pour la description d'une aberration de front d'onde mesurée avec l'accommodation de l'oeil en vision de près sur une deuxième position de mesure ;

   et où la détermination de données de réfraction objectives de l'oeil pour une vision de près comprend :

   - la détermination du premier vecteur de puissance $\mathbf{P}_F(VD_F) = \begin{pmatrix} M_F(VD_F) \\ \mathbf{J}_F(VD_F) \end{pmatrix}$ à partir du premier ensemble de polynômes de Zernike compris dans les premières données de mesure ou ajusté selon l'une des revendications 3 à 5 en fonction de la distance $VD_F$ d'une position d'évaluation à la première position de mesure, de sorte que $M_F(VD_F)$ décrive l'équivalent sphérique et $\mathbf{J}_F(VD_F)$ la part cylindrique de l'aberration de front d'onde sur la position d'évaluation avec l'accommodation de l'oeil en vision de loin ; et

   - la détermination du deuxième vecteur de puissance $\mathbf{P}_N(VD_N) = \begin{pmatrix} M_N(VD_N) \\ \mathbf{J}_N(VD_N) \end{pmatrix}$ à partir du deuxième ensemble de polynômes de Zernike compris dans les deuxièmes données de mesure ou ajusté selon l'une des revendications 3 à 5 en fonction de la distance $VD_N$ de la position d'évaluation à la deuxième position de mesure de sorte que $M_N(VD_N)$ décrive l'équivalent sphérique et $\mathbf{J}_N(VD_N)$ la part cylindrique de l'aberration de front d'onde sur la position d'évaluation avec l'accommodation de l'oeil en vision de près.

7. Procédé selon la revendication 6, où le premier vecteur de puissance et/ou le deuxième vecteur de puissance sont déterminés de de manière à satisfaire à l'égalité

$$\mathbf{P}_i(VD_i) = \begin{pmatrix} Sph_i(VD_i) + \dfrac{Cyl_i(VD_i)}{2} \\ -\dfrac{Cyl_i(VD_i)}{2}\cos 2Axis_i(VD_i) \\ -\dfrac{Cyl_i(VD_i)}{2}\sin 2Axis_i(VD_i) \end{pmatrix}$$

avec

$$Sph_i(VD_i) = \frac{Sph_i(0)}{1 + VD_i \times Sph_i(0)}$$

$$Cyl_i(VD_i) = \frac{Sph_i(0) + Cyl_i(0)}{1 + VD_i \times (Sph_i(0) + Cyl_i(0))} - \frac{Sph_i(0)}{1 + VD_i \times Sph_i(0)}$$

$$Axis_i(VD_i) = Axis_i(0)$$

et

$$Sph_i(0) = -\frac{4\sqrt{3}}{r_0^2} c_{2,i}^0 + \frac{2\sqrt{6}}{r_0^2} \sqrt{\left(c_{2,i}^{-2}\right)^2 + \left(c_{2,i}^2\right)^2}$$

$$Cyl_i(0) = -\frac{4\sqrt{6}}{r_0^2} \sqrt{\left(c_{2,i}^{-2}\right)^2 + \left(c_{2,i}^2\right)^2}$$

$$Axis_i(0) = \frac{1}{2}\arctan\left(c_{2,i}^2, c_{2,i}^{-2}\right) + \frac{\pi}{2}$$

pour $i = F, N$, où

$$\arctan(x, y) := \begin{cases} \arctan(y/x), & x > 0 \\ \arctan(y/x) + \pi, & x < 0, y > 0 \\ \arctan(y/x) - \pi, & x < 0, y < 0 \\ \pi/2, & x = 0, y > 0 \\ -\pi/2, & x = 0, y < 0 \end{cases}.$$

et où $c_{2,i}^0$, $c_{2,i}^2$ et $c_{2,i}^{-2}$ désignent les polynômes de Zernike de deuxième ordre ajustés compris dans les premières ($i = F$) ou les deuxièmes ($i = N$) données de mesure ou selon l'une des revendications 3 à 5 pour la vision de loin ($i = F$) ou la vision de près ($i = N$).

8. Procédé selon l'une des revendications précédentes, où la saisie des premières et/ou des deuxièmes données de mesure comprennent une mesure des données de réfraction de l'oeil au moyen d'un autoréfracteur et/ou d'un aberromètre.

9. Dispositif pour la détermination de réfraction objective pour un oeil d'un porteur de lunettes, comprenant une interface de saisie de données de mesure pour la saisie de données de mesure pour l'oeil du porteur de lunettes, lesquelles définissent au moins un premier ensemble de polynômes de Zernike $c_{2,F}^0$, $c_{2,F}^2$ et $c_{2,F}^{-2}$ pour la description d'une aberration de front d'onde avec une accommodation en vision de loin et un rayon de pupille photopique $r_0$ de l'oeil, et un deuxième ensemble de polynômes de Zernike $c_{2,N}^0$, $c_{2,N}^2$ et $c_{2,N}^{-2}$ pour la description d'une aberration de front d'onde avec une accommodation en vision de près et le rayon de pupille photopique $r_0$ de l'oeil ; ledit dispositif étant **caractérisé en ce qu'**il comprend en outre :

- un dispositif de détermination de données de réfraction pour la détermination de données de réfraction objectives pour la sphère ($Sph_N^{corr}$), le cylindre ($Cyl_N^{corr}$) et la position d'axe ($Axis_N^{corr}$) de l'oeil pour une vision de près, de sorte qu'en fonction du premier et du deuxième ensemble de polynômes de Zernike, les données de réfraction objectives satisfassent aux égalités

$$Sph_N^{corr} = M_N^{corr} + \sqrt{\left(J_{N,0}^{corr}\right)^2 + \left(J_{N,45}^{corr}\right)^2}$$

$$Cyl_N^{corr} = -2\sqrt{\left(J_{N,0}^{corr}\right)^2 + \left(J_{N,45}^{corr}\right)^2}$$

$$Axis_N^{corr} = \frac{1}{2}\arctan\left(-J_{N,0}^{corr}, -J_{N,45}^{corr}\right) + \frac{\pi}{2}$$

pour un vecteur de puissance corrigé $\mathbf{P}_N^{corr} = \begin{pmatrix} M_N^{corr} \\ J_{N,0}^{corr} \\ J_{N,45}^{corr} \end{pmatrix}$ pour la vision de près, où, en fonction d'une différence

$\Delta\mathbf{P} = \begin{pmatrix} \Delta M \\ \Delta\mathbf{J} \end{pmatrix} = \mathbf{P}_N - \mathbf{P}_F$ entre un premier vecteur de puissance

$$\mathbf{P}_F = \begin{pmatrix} M_F \\ \mathbf{J}_F \end{pmatrix} = \begin{pmatrix} -\dfrac{4\sqrt{3}}{r_0^2}c_{2,F}^0 \\ -\dfrac{2\sqrt{6}}{r_0^2}c_{2,F}^2 \\ -\dfrac{2\sqrt{6}}{r_0^2}c_{2,F}^{-2} \end{pmatrix}$$

et un deuxième vecteur de puissance

$$\mathbf{P}_N = \begin{pmatrix} M_N \\ \mathbf{J}_N \end{pmatrix} = \begin{pmatrix} -\dfrac{4\sqrt{3}}{r_0^2}c_{2,N}^0 \\ -\dfrac{2\sqrt{6}}{r_0^2}c_{2,N}^2 \\ -\dfrac{2\sqrt{6}}{r_0^2}c_{2,N}^{-2} \end{pmatrix},$$

le vecteur de puissance $P_N^{corr}$

- correspond à la valeur $\mathbf{P}_N - \begin{pmatrix} A_{1N} \\ 0 \\ 0 \end{pmatrix}$, si $\Delta M > A_{1N}$ ; et

- correspond à la valeur $\mathbf{P}_F + \dfrac{A_{1N}}{\Delta M}\begin{pmatrix} 0 \\ \Delta\mathbf{J} \end{pmatrix}$, si $\Delta M \leq A_{1N}$, où $A_{1N} = -\dfrac{1}{d}$ est l'équivalent sphérique d'une distance d'objet définie $d$ pour la vision de près, et où

$$\arctan(x,y) := \begin{cases} \arctan(y/x), & x > 0 \\ \arctan(y/x) + \pi, & x < 0, y > 0 \\ \arctan(y/x) - \pi, & x < 0, y < 0 \\ \pi/2, & x = 0, y > 0 \\ -\pi/2, & x = 0, y < 0 \end{cases}.$$

**10.** Produit de programme informatique comprenant des instructions lisibles par ordinateur, qui, chargées en mémoire d'un ordinateur et exécutées par celui-ci, entraînent l'exécution par l'ordinateur d'un procédé selon l'une des reven-

dications 1 à 8.

Fig. 1

Fig. 2

ST1A

ST2A

ST1B

ST2B

ST1C

ST2C

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2005058136 A2

**In der Beschreibung aufgeführte Nicht-Patentliteratur**

- **PORTER et al.** Adaptive Optics for Vision Science. Wiley, 2006, 334